# EUROPÄISCHE PATENTSCHRIFT

(11) **EP 1 575 833 B1**
(45) Veröffentlichungstag und Bekanntmachung des Hinweises auf die Patenterteilung: **07.05.2008**
(21) Anmeldenummer: 03788803.9
(22) Anmeldetag: 21.11.2003
(51) Int. Cl.: B65D 1/00

(54) **VERFAHREN ZUR HERSTELLUNG UND/ODER HANDHABUNG EINES HOCHREINEN GEGENSTANDES**
METHOD FOR THE PRODUCTION AND/OR HANDLING OF A HIGHLY PURE OBJECT
PROCEDE DE FABRICATION ET / OU DE MANIPULATION D'UN OBJET A DEGRE DE PROPRETE TRES ELEVE

(30) Priorität: 22.11.2002 DE 10254762
(43) Veröffentlichungstag der Anmeldung: 21.09.2005
(73) Patentinhaber: Transcoject GmbH & Co. KG, 24539 Neumünster (DE)
(72) Erfinder: HEINZ, Jochen, 24220 Flintbek (DE)
(74) Vertreter: Hemmer, Arnd
(86) Internationale Anmeldenummer: PCT/DE2003/003861
(87) Internationale Veröffentlichungsnummer: WO 2004/048207

(56) Entgegenhaltungen:
- EP-A- 0 246 587
- EP-A- 0 849 173
- DE-A- 10 050 660
- US-A- 4 037 830
- US-A- 5 687 542

## Beschreibung

Die Erfindung betrifft ein Verfahren zur Herstellung und/oder Handhabung eines hochreinen Gegenstandes, insbesondere eines medizinischen Behälters, beispielsweise eines vorfüllbaren Behälters zur Aufnahme von Arzneimitteln. Ferner betrifft die Erfindung eine entsprechende Vorrichtung zur Handhabung eines solchen hochreinen Gegenstandes.

Es sind medizinische Behälter bekannt, welche zur Aufbewahrung von Substanzen für Medizin und Pharmazie verwendet werden. Solche Behälter sind insbesondere vorfüllbare Behälter wie z.B. vorfüllbare Flaschen oder vorfüllbare Spritzen aus Glas oder auch Kunststoff, welche mit einem Arzneimittel vorgefüllt ausgeliefert wird.

Derartige Behälter zur Aufbewahrung von Substanzen für Medizin und Pharmazie müssen im Wesentlichen zwei Aspekte erfüllen, nämlich die aufzubewahrende Substanz vor Veränderungen zu schützen und zum anderen den Inhalt des Behälters vor Verunreinigungen zu bewahren. Die behördlichen Mindestanforderungen dazu sind beispielsweise in den Arzneibüchern beschrieben und somit zwingend vorgeschrieben. Im Einzelnen können Produktanforderungen noch weit darüber hinausgehen.

Mögliche Verunreinigungen wie Partikel oder Keime können nicht nur nachträglich aus der Umgebung in den Behälter eingebracht werden, vielmehr können sie auch aus dem Behälter selbst stammen, d. h. beispielsweise im oder durch den Produktionsprozess des Behälters in bzw. an diesen gelangt sein. Daher schreiben die einschlägigen Vorschriften Höchstwerte für zulässige Partikel- und Endotoxinlasten vor.

Insbesondere kann eine Verunreinigung von Kunststoffortikeln dadurch auftreten, dass sie nach dem Herstellungs- und Entformungsprozess eine elektrostatische Aufladung aufweisen, welche Partikel aus der Umgebungsluft anzieht und darüber hinaus ein Abspülen anhaftender Partikel verhindert. Daher werden in üblichen Herstellungsprozessen Verfahren eingesetzt, um die Kunststoffteile nach der Entformung zu entladen. Dabei erfolgt die Entladung jedoch häufig unvollständig und es treten Nachladungseffekte auf, bei denen Ladungen aus dem Inneren der Kunststoffteile über längere Zeit an die Oberfläche gelangen.

Üblicherweise werden Partikel- und Endotoxinlasten dadurch verhindert, dass die Behälter vor dem Befüllen gewaschen werden, wie es beispielsweise in US 4,718,463 beschrieben ist. Darüber hinaus werden diese Behältnisse in der Regel durch die Anwendung hoher Temperaturen bis zu 300 ° Celsius entpyrogenisiert. Diese Anwendung von hohen Temperaturen kann jedoch nur bei Behältnissen aus Glas eingesetzt werden, da Behältnisse aus Kunststoff in der Regel bei diesen Temperaturen zerstört würden.

Daher werden zur Herstellung und Reinigung von Kunststoffbehältern andere Verfahren angewendet. So beschreibt US 5,620,425 die Herstellung eines vorfüllbaren Spritzenzylinders in einem Reinraum der Klasse 100, wodurch Verunreinigungen während der Herstellung des Spritzenkörpers vermieden werden sollen. Die vollständige Produktion eines Spritzenkörpers bzw. einer Spritze in einem Reinraum ist jedoch nur sehr aufwändig möglich. So lässt sich eine Reinraumatmosphäre der Klasse 100 nur über einen Laminar-Flow erzeugen, welcher jedoch in einer Spritzgussmaschine durch die Öffnungs- und Schließbewegung der Maschine nicht oder nur schwerlich aufrechtzuerhalten ist und auch durch in dem Reinraum arbeitenden Personen leicht gestört wird. Daher sind die in US 5,620,425 beschriebenen Bedingungen bei der Herstellung einer Kunststoffspritze im Spritzguss gar nicht oder nur sehr schwer aufrechtzuerhalten, um die geforderte Keimfreiheit zu erzielen. Hinzu kommt, dass die Reinraumbedingungen und deren Tauglichkeit für das jeweilige Produkt zunächst aufwändig validiert und dann im Betrieb intensiv überwacht werden müssen. Insgesamt stellt also das Betreiben solcher Reinräume einen erheblichen Aufwand dar, welcher zu einer erheblichen Verteuerung der hergestellten Produkte führt.

US 6,164,044, US 6,189,292, US 6,263,641 und US 6,250,052 beschreiben daher ein weiteres Herstellungsverfahren zur Herstellung von vorfüllbaren Behältern aus Glas oder Kunststoff. Gemäß den in diesen Patenten beschriebenen Verfahren werden die Behälter bzw. Spritzenzylinder nach der Herstellung durch Gießen oder Umformen von Glas oder Spritzgießen von Kunststoff zur Weiterverarbeitung in ein abgeschlossenes System verbracht. Dieses System besteht aus einzelnen Containern bzw. Schränken, in welchen eine Reinraumatmosphäre herrscht. Wenn die außerhalb dieser Reinraumatmosphäre gefertigten Behälter in das abgeschlossene System gebracht werden, werden sie zunächst durch einen Strom gereinigter Luft gereinigt, so dass eventuell anhaftende Partikel oder Keime von den Behältnissen abgespült werden. Anschließend werden die so gereinigten Behältnisse in dem System, in welchem Reinraumbedingungen der Klasse 100 herrschen, weiter verarbeitet.

Auch diese Anlage hat den Nachteil, dass für die gesamte Handhabung und das Befüllen in den geschlossenen Schränken bzw. Containern Reinraumbedingungen der Klasse 100 geschaffen werden müssen. Ferner besteht die Gefahr, dass trotz der anfänglichen Reinigung der außerhalb des Reinraumsystems gefertigten Behältnisse an diesen Keime oder Partikel anhaften.

US 5,687,542 offenbart eine Vorrichtung gemäß dem Oberbegriff des Anspruchs 25 zur Fertigung beispielsweise von Spritzen. Bei dieser Vorrichtung ist eine Spritzgussmaschine vorgesehen und eine direkt angrenzende Bearbeitungsstation, innerhalb derer bessere Reinraumbedingungen als in der Umgebung herrschen. Nach dem Spritzgießen des Gegenstandes wird das Werkzeug geöffnet und der Gegenstand direkt in die angrenzende Bearbeitungsstation hineinbewegt. Dabei wird aus der Bearbeitungsstation hochreine Luft in Richtung des Werkzeuges geleitet, um das Eindringen von Verunreinigungen in die Bearbeitungsstation zu verhindern.

Es ist Aufgabe der Erfindung, ein neues Verfahren sowie eine Vorrichtung zur Herstellung und/oder Handhabung eines heißgeformten, anfänglich hochreinen Gegenstandes wie eines medizinischen Behälters zu schaffen, welche eine kostengünstige und einfache Fertigung ermöglichen und gleichzeitig einen größere Reinheit gewährleisten können. Insbesondere soll ein effizienteres Verfahren zur Herstellung medizinischer Behälter geschaffen werden, welches die Anforderungen der Arzneibücher in Bezug auf Sauberkeit, insbesondere in Bezug auf Partikel und/oder Endotoxine erfüllt und übertrifft und auf die Anwendung sehr sauberer Reinräume insbesondere der Klasse 100 verzichten kann.

Diese Aufgabe wird durch ein Verfahren mit den in Anspruch 1 angegebenen Merkmalen sowie durch eine Vorrichtung mit den in Anspruch 25 angegebenen Merkmalen gelöst. Bevorzugte Ausführungsformen ergeben sich aus den Unteransprüchen.

Das erfindungsgemäße Verfahren bezieht sich auf die Herstellung und/oder Handhabung eines hochreinen Gegenstandes. Bei einem solchen Gegenstand kann es sich beispielsweise um einen medizinischen oder medizintechnischen Gegenstand handeln, welcher hochrein sein muss, dass heißt im Wesentlichen frei von Keimen und Partikeln. Gemäß dem Verfahren wird der hochreine Gegenstand während eines Handhabungsvorganges durch ein Fluid, welches den Gegenstand umströmt bzw. umgibt, von der Umgebung abgeschirmt. Dabei werden während des gesamten Handhabungsvorganges zumindest die Teile des Gegenstandes ständig von dem Fluid umströmt, welche die geforderte Reinheit aufweisen müssen. So werden diese Teile ständig ein einer definierten schützenden Atmosphäre gehalten. Dadurch wird erreicht, dass ein anfänglich hochreiner Gegenstand während der Handhabung und weiteren Verarbeitung nicht durch Kontakt mit der Umgebungsluft verunreinigt wird. Somit kann auf eine besondere Reinraumumgebung und/oder spätere Reinigungsschritte verzichtet werden, wodurch das Herstellungsverfahren vereinfacht wird. Ferner kann eine größere Reinheit sichergestellt werden, da Verunreinigungen des Gegenstandes von Anfang an verhindert werden können anstatt sie in späteren Reinigungsschritten wieder zu beseitigen, wobei ein vollständiges Entfernen von Verunreinigungen bei der Reinigung meist nicht möglich ist. Ferner hat das Verfahren gegenüber bekannten Verfahren, bei denen der Gegenstand kurzeitig zur Reinigung mit einem Fluid gespült wird, den Vorteil, dass geringere Strömungsgeschwindigkeiten des Fluids und geringere Fluidmengen zur Abschirmung des Gegenstandes ausreichend sind. Ferner bewirkt der Wegfall eines Reinigungsschrittes eine Verkürzung des gesamten Herstellungsverfahrens, was neben einer höheren Effizienz des Verfahrens eine Verringerung des Risikos von Verunreinigungen des Gegenstandes bewirkt. Durch die direkte Abschirmung des Gegenstandes durch das umgebende Fluid während des Herstellungsprozesses und während der Handhabung können Transferschritte zwischen verschiedenen Umgebungen vermieden werden. Der Gegenstand bleibt immer in der von dem umströmenden Fluid erzeugten Umgebung.

Bei dem Gegenstand handelt es sich um einen in einem Werkzeug heißgeformten Gegenstand, wobei der Gegenstand während des gesamten Entnahmevorganges aus dem Werkzeug durch das umströmende Fluid von der Umgebung abgeschirmt wird. Der Gegenstand ist beispielsweise ein Gegenstand aus Metall oder Kunststoff, welcher im Gussverfahren, z.B. Spritz- oder Druckguss in dem Werkzeug gefertigt worden ist. Dabei nutzt die Erfindung den Effekt aus, dass ein heißgeformter, z.B. aus schmelzflüssigen Kunststoff hergestellter Gegenstand nach dem Erstarren eine perfekte Sauberkeit aufweist. Dies gilt speziell in Bezug auf Partikel und aufgrund der Schmelztemperaturen bis zu mehr als 300° Celsius auch in Bezug auf Endotoxine. Durch das Umströmen des frisch geformten Gegenstandes während der Entnahme aus dem Werkzeug wird verhindert, dass der aufgrund des Herstellungsprozesses perfekt saubere Gegenstand nachträglich verschmutzt wird. Der Gegenstand kommt aufgrund des Umströmens bzw. Umhüllens mit einem Fluid überhaupt nicht mit der Umgebungsluft in Kontakt, so dass eine Verschmutzung des Gegenstandes von Anfang an verhindert wird. Dies hat den Vorteil, dass keine besonders reinen Umgebungsbedingungen geschaffen werden müssen, so kann bei der Herstellung medizinischer Gegenstände bzw. Behälter beispielsweise auf teure und aufwändige Reinräume der Klasse 100 verzichtet werden. Da erfindungsgemäß von Anfang an eine Verschmutzung des Gegenstandes verhindert wird, ist es auch nicht wie beim Stand der Technik erforderlich, den Gegenstand vor der Weiterverarbeitung durch eine Luftdusche oder ähnliches zu reinigen. Erfindungsgemäß kann der reine, durch das umhüllende Fluid vor Verschmutzung geschützte Gegenstand ohne Zwischenschritt direkt in eine Weiterverarbeitung gegeben werden. Somit kann insgesamt ein sehr kostengünstiger und effektiver Herstellungsprozess geschaffen werden.

Das Verfahren eignet sich besonders bevorzugt zur Herstellung eines Gegenstandes, welcher Teil eines medizinischen Behälters oder ein medizinischer Behälter ist. Bei einem solchen Behälter kann es sich beispielsweise um ein vorfüllbares Fläschchen oder eine vorfüllbare Spritze aus einem geeigneten Kunststoff insbesondere einem Barrierekunststoff handeln, welche in dem Werkzeug geformt werden. Das Formen des Behälterteils bzw. Behälters erfolgt vorzugsweise im Spritzguss- oder Spritzblasverfahren. Gemäß dem erfindungsgemäßen Verfahren können alle Teile bzw. Komponenten eines medizinischen Behälters, insbesondere solche Teile, welche mit einem Arzneimittel in Kontakt kommen, gefertigt und gehandhabt werden, ohne im Anschluss an den Formprozess zu verschmutzen. Dabei wird durch die Abschirmung mittels des Fluids erreicht, dass es nicht erforderlich ist, den Behälter vor dem Befüllen nochmals zu reinigen oder zu spülen. Die ursprünglich gegebene Reinheit bzw. Sterilität bei der Entnahme aus dem Werkzeug wird bis zum Befüllen aufrechterhalten, ohne dass der Handhabungsprozess in einem besonderen Reinraum der Klasse 100 ablaufen muss.

Vorzugsweise handelt es sich bei dem Fluid, mit dem der Gegenstand umströmt wird, um ein Gas, insbesondere Luft oder gefilterte Luft. Durch die Filterung kann die erforderlich Keim- und Partikelfreiheit des Gases bzw. der Luft sichergestellt werden. Vorzugsweise werden 0,2 µm-Filter oder Filter mit noch kleineren Porendurchmessern eingesetzt, um die erforderliche Sauberkeit der Luft sicherzustellen. Die Luft bzw. gefilterte Luft umgibt den Gegenstand möglichst vollständig, so dass eine Lufthülle geschaffen wird, welche den aufgrund des vorangehenden Fertigungsprozesses sauberen Gegenstand vor der möglicherweise verunreinigten Umgebungsluft schützt.

Weiter bevorzugt ist das Fluid, von dem der Gegenstand umströmt wird, konditionierte Luft. Die Luft kann beispielsweise angefeuchtet sein, um bei der Entnahme des Gegenstandes, z.B. eines Behälterteils aus dem Werkzeug statische Aufladungen zu verhindern bzw. zu kompensieren. Statische Aufladungen des Gegenstandes werden durch den direkten Einsatz der konditionierten Luft bei der Entnahme des Gegenstandes aus dem Werkzeug von vornherein vermieden, so dass ein Anhaften von Partikeln oder Keimen aufgrund statischer Aufladung verhindert werden kann. Vorzugsweise wird bei der Entnahme eines Behälterteils bzw. Behälters aus dem Werkzeug der in dem Behälterteil beim Entformen des Kerns entstehende Hohlraum direkt mit dem umströmenden Gas, insbesondere gefilterter und/oder konditionierter Luft belüftet.

Noch weiter bevorzugt handelt es sich bei dem Fluid, von dem der Gegenstand umströmt wird, um ionisierte Luft. Dabei kann es sich um gefilterte, konditionierte und ionisierte Luft handeln. Auf diese Weise kommt der zu handhabende Gegenstand nur mit der in dieser Weise aufbereiteten Luft in Kontakt und eine ggf. beim Entnahmeprozess durch Reibung entstehende elektrostatische Aufladung kann in statu nascendi, d.h. direkt bei der Entstehung kompensiert werden. Auch können, da keine Ladungen mehr entstehen, diese auch nicht mehr in das Innere einer Kunststoffmatrix gelangen, was gemeinsam mit dem unten Beschriebenen Nachladungseffekten, wie sie bei bekannten Verfahren auftreten, entgegenwirkt. Ferner bewirkt das Umströmen des Gegenstandes, dass der Gegenstand sehr lange mit dem Fluid bzw. Gas bzw. der aufbereiteten Luft in Kontakt ist. Dies hat gegenüber bekannten Luftduschen oder Luftvorhängen, durch die ein Gegenstand bzw. Behälterteil zur Reinigung geführt wird oder gravitationsbedingt hindurch fällt, den Vorteil, dass mit relativ geringen Entladeströmen gearbeitet werden kann und Nachladungseffekte, wie sie beim Stand der Technik auftreten, kompensiert werden. Bevorzugt kann ferner die Ladung des Gegenstandes gemessen werden und die Strömung von ionisierter Luft so gesteuert bzw. geregelt werden, dass die in dem Gegenstand auftretenden Ladung genau kompensiert wird, ohne dass es zu einer erneuten unerwünschten Ladung kommt. Zusätzlich können die den Gegenstand haltenden Greifer geerdet sein, um Ladungen abzuführen.

Das Fluid, von dem der Gegenstandumströmt wird, kann ferner bevorzugt zumindest als Bestandteil ein keimtötendes Fluid bzw. Gas enthalten. So kann durch Verwendung eines keimtötenden Fluids oder Beimengung keimtötender Stoffe in das Fluid bzw. Gas zusätzlich ein Abtöten von Keimen erfolgen, welche sich in der Umgebungsluft befinden. Als keimtötendes Gas kann beispielsweise ein H₂O₂-haltiges Gas oder Ozon o.ä. verwendet werden. Alternativ zu keimtötenden Gas können, wie bereits beschrieben, gereinigte Luft, CO₂, Edelgase oder andere Gase zum Umströmen bzw. Umhüllen des Gegenstandes insbesondere bei der Entnahme aus dem Werkzeug eingesetzt werden. Es können alle geeigneten Gase eingesetzt werden, welche eine hochreine Atmosphäre in direkter Umgebung des Gegenstandes schaffen, um eine Verunreinigung durch die Umgebungsluft zu verhindern.

Die Umströmung des Gegenstandes beginnt zweckmäßigerweise, wenn sich der Gegenstand noch in dem Werkzeug befindet. Besonders bevorzugt beginnt die Umströmung bzw. Umhüllung des Gegenstandes direkt nach Öffnen des Werkzeuges, so dass der so gefertigte Gegenstand überhaupt nicht mit der Umgebungsluft in Kontakt kommt. Auf diese Weise kann eine Verunreinigung des steril bzw. sauber gefertigten Gegenstandes beim Öffnen des Werkzeuges und bei der Entnahme sowie der weiteren Verarbeitung sicher verhindert werden.

Bevorzugt erfolgt die Entnahme des Gegenstandes aus dem Werkzeug in definierter Weise maschinell. Durch die maschinelle Entnahme kann der Gegenstand in vordefinierter Weise und mit vorbestimmter Geschwindigkeit aus dem Werkzeug entnommen werden. Dadurch kann erreicht werden, dass immer eine Geschwindigkeit eingehalten wird, bei der sichergestellt ist, dass die Hülle aus dem den Gegenstand umströmenden Fluid bzw. Gas nicht verweht oder beschädigt wird. So ist auch während der Bewegung des Gegenstandes bei der Entnahme sichergestellt, dass dieser durch das Fluid von der Umgebungsluft abgeschirmt ist. Ferner kann durch die definierte Bewegung die statische Aufladung beim Herausnehmen des Gegenstandes aus dem Werkzeug minimiert werden. Auch kann der Bewegungsablauf des Gegenstandes zu dem Werkzeug bei der maschinellen Entnahme so gesteuert werden, dass möglichst keine Partikel beim Entformen des Gegenstandes gebildet werden, z.B. aufgrund von Reibung zwischen Werkzeug und Gegenstand. Die definierte maschinelle Entnahme aus dem Werkzeug kann beispielsweise durch einen Roboterarm oder eine andere geeignete Handhabungseinrichtung erfolgen, welche mit vorbestimmten Geschwindigkeiten und Beschleunigungen betrieben werden kann.

Besonders bevorzugt wird der Gegenstand aus dem Werkzeug durch einen Roboter entnommen und gleichzeitig durch einen in dem Werkzeug angeordneten Auswerfer von dem Werkzeug getrennt bzw. ausgeworfen. Dies ermöglicht die Entnahme eines Kunststoffgegenstandes in noch relativ weichem Zustand. Durch den Auswerfer und den den Gegenstand greifenden Roboter wird die erforderliche Entnahme- bzw. Trennkraft, um den Gegenstand aus dem Werkzeug zu entnehmen, an mehreren Stellen auf den Gegenstand aufgebracht. Das Material des Gegenstandes muss somit bei der Entnahme nur geringere Kräfte übertragen. Dadurch werden punktuell einwirkende hohe Kräfte, welche zu Verformungen des noch weichen Gegenstandes führen könnten, vermieden.

Vorzugsweise erfolgt die Entnahme des Gegenstandes aus dem Werkzeug mit einer geringen Anfangsgeschwindigkeit. Das bedeutet, der Gegenstand wird zunächst mit einer möglichst geringen Geschwindigkeit von dem Werkzeug gelöst. Anschließend kann die Bewegungsgeschwindigkeit schrittweise oder progressiv erhöht werden, um eine schnelle Handhabung zu ermöglichen. Durch die geringe Anfangsgeschwindigkeit kann eine saubere Trennung des Gegenstandes von der Werkzeugoberfläche erreicht werden, ohne das entformungsbedingte Partikel an der Oberfläche des Gegenstandes haften bleiben. Mögliche Verunreinigungen des Gegenstandes während des Entnahmevorgangs aus dem Werkzeug werden somit weiter minimiert.

Die Entnahme des Gegenstandes aus dem Werkzeug erfolgt vorzugsweise vor dem vollständigen Abkühlen des Gegenstandes. Die Entnahme des Gegenstandes erfolgt bei einer möglichst hohen Entnahmetemperatur, welche einen noch relativ weichen Kunststoff zur Folge hat. Auch hierbei ist die definierte maschinelle Entnahme von Vorteil, da nur eine solche eine verformungsfreie Entnahme bei noch weichem Kunststoff ermöglicht im Gegensatz zu einer ausschließlich werkzeuggebundenen Entformung des Kunststoffartikels. Der noch weiche Kunststoff ermöglicht ein sauberes Ablösen von der Werkzeugoberfläche, ohne dass unerwünschte Partikel entstehen, da die Oberfläche des Kunststoffes auf mikroskopischer Ebene noch eine gewisse Plastizität aufweist. Ferner können statische Aufladungen aufgrund von Reibung minimiert werden. Das den Gegenstand bei der Entnahme umspülende Fluid sorgt dann für eine gezielte Abkühlung.

Die Entnahme des Gegenstandes aus dem Werkzeug erfolgt gemäß einer bevorzugten Ausführungsform durch einen Roboter und an dem Roboter ist mindestens eine Düse angeordnet, durch welche der Gegenstand mit dem Fluid umströmt wird. Dabei ist die Düse bzw. sind die Düsen möglichst nahe an einer Greifeinrichtung des Roboterarmes angeordnet, welche den Gegenstand ergreifen. Durch diese Anordnung wird sichergestellt, dass während des gesamten Bewegungsvorganges des Gegenstandes durch den Roboter der Gegenstand von dem Fluid umströmt bzw. umhüllt wird, so dass der Gegenstand gegenüber der Umgebungsluft abgeschirmt wird. Dabei wird der Gegenstand möglichst dicht umströmt, um die Ausdehnung der von dem Fluid bzw. Gas erzeugten Atmosphäre und damit die erforderliche Fluidmenge möglichst gering zu halten.

Zusätzlich können in zumindest einem Teil des Werkzeuges Düsen zum Umströmen des Gegenstandes mit dem Fluid angeordnet sein. Durch diese Düsen kann gewährleistet werden, dass der Gegenstand bereits im Werkzeug direkt beim Öffnen des Werkzeuges umströmt wird, so dass er während des gesamten Entnahmeprozesses aus dem Werkzeug nicht mit der Umgebungsluft in Kontakt kommt. Dabei können die Düsen für das Fluid im beweglichen und/oder festen Teil des Werkzeuges angebracht sein. Die genaue Anordnung hängt von der Geometrie des Werkzeuges und des zu erzeugenden Bauteils ab. Die Düsen werden so angeordnet, dass bei der Entnahme das Bauteil bzw. Behälterteil ständig von Fluid bzw. Gas, insbesondere hochreiner Luft umströmt ist, um eine Kontamination mit Verschmutzungen aus der Umgebung zu verhindern.

Das Werkzeug weist vorzugsweise eine Oberfläche auf, welche derart behandelt ist, dass sie ein minimales Haftvermögen aufweist. Auch dies trägt dazu bei, dass beim Entformen keine unerwünschten Partikel entstehen, welche möglicherweise an der Oberfläche des Gegenstandes anhaften können. So wird von vornherein ein ausreichend sauberer Gegenstand geschaffen, welcher keiner anschließenden Reinigung mehr bedarf, da er erfindungsgemäß während des gesamten Prozesses durch ein umströmendes Fluid von der Umgebungsluft abgeschirmt wird. Die Oberfläche des Werkzeuges wird vorzugsweise mit einer nicht zu geringen und einer nicht zu großen Rauheit ausgebildet, um eine möglichst minimale Haftung zwischen Gegenstand und Werkzeug zu erreichen. Zusätzlich kann die Oberfläche des Werkzeuges mit geeigneten Materialien wie beispielsweise Teflon oder Titannitrid beschichtet werden. Auch alle anderen geeigneten Beschichtungen oder Verfahren zur Behandlung der Werkzeugoberfläche können eingesetzt werden, um eine minimale Anhaftung zwischen dem erzeugten Gegenstand und dem Werkzeug zu realisieren.

Zusätzlich zu dem umströmenden Fluid kann der Gegenstand direkt bei der Entnahme aus dem Werkzeug zusätzlich zur Umströmung mit dem Fluid von einer Schutzglocke umgeben werden. Eine derartige Schutzglocke ist ein zumindest einseitig offener Hohlkörper, so dass durch die Öffnung der Gegenstand in die Glocke gelangen kann. Die Glocke kann beispielsweise aus Kunststoff oder Metall bestehen und ist vorzugsweise an einem Roboterarm angebracht, welcher den Gegenstand aus dem Werkzeug entnimmt und weiter handhabt. Dabei wird das den Gegenstand umströmende Fluid, insbesondere ein Gas vorzugsweise so geleitet, dass es die Glocke vollständig füllt, so dass keine möglicherweise verunreinigte Umgebungsluft in die Glocke gelangt. Die Glocke hat den Vorteil, dass auch bei einer schnellen Bewegung des Behälterteils durch den Roboterarm ein Verwehen der den Gegenstand umgebenden Fluid- bzw. Gasschichten sicher verhindert wird. So kann bei Bewegung des Gegenstandes jederzeit eine ausreichende Abschirmung von der Umgebungsluft sichergestellt werden.

An die Entnahme des Gegenstandes aus dem Werkzeug schließt sich vorzugsweise eine automatische oder halbautomatische Weiterverarbeitung an. Diese kann einen oder mehrere Weiterverarbeitungsschritte wie zum Beispiel im Falle eines medizinischen Behälters bzw. Behälterteils eine Silikonisierung, Inspizierung, Montage, Kennzeichnung, Befüllen, Verpackung etc. beinhalten. Dabei kann diese Weiterverarbeitung in einer abgeschlossenen Anlage erfolgen, in der ausreichende Reinraumbedingungen herrschen, wie beispielsweise aus US 6,189,292, US 6,263,641, US 6,250,052 und US 6,164,044 bekannt ist. Dadurch, dass er findungsgemäß ursprünglich saubere Teile in die Weiterverarbeitung gegeben werden, wird eine größere Freiheit im nachfolgenden Prozess erreicht, da die für die Verunreinigung zulässigen Toleranzen weniger weit ausgeschöpft sind.

Die Abschirmung des aus dem Werkzeug entnommenen Gegenstandes durch das umströmende Fluid wird auch bei zumindest einem nachfolgenden Handhabungs- und/oder Verarbeitungsschritten aufrechterhalten. So kann auch bei diesen nachfolgenden Handhabungs- und/oder Verarbeitungsschritten auf eine Reinraumumgebung, insbesondere eine Reinraumumgebung der Klasse 100 verzichtet werden, da der Gegenstand, bevorzugt ein Behälterteil, ständig durch die Umhüllung bzw. das Umströmen mit dem Fluid von der Umgebungsluft abgeschirmt wird. Dabei bildet das umgebende Fluid eine stetig aufrechterhaltende Hülle um den Gegenstand, welche eine Verschmutzung verhindert. Um diese Fluidhülle, insbesondere aus hochreiner Luft aufrechterhalten zu können, werden entsprechende Luftdüsen mit dem Produkt bzw. dem Behälterteil mitgeführt. Vorzugsweise sind die erforderlichen Düsen direkt an einem Roboterarm, welcher den Gegenstand bewegt, angebracht. Dadurch, dass der Gegenstand während des gesamten Prozessen in der schützenden Fluidhülle gehalten wird, werden Transfers zwischen verschiedenen Umgebungen durch entsprechende Schleusen überflüssig, wodurch das Verfahren einfacher und sicherer wird.

Das Umströmen des aus dem Werkzeug entnommenen Gegenstandes mit dem Fluid kann zur raschen Abkühlung des Behälterteils benutzt werden. Zum Beispiel bei teilkristallinen Kunststoffen oder zur Verhinderung der Kristallisation kann eine gezielte schnelle Abkühlung des Gegenstandes gewünscht sein. Durch entsprechende Temperierung des Fluids, mit welchem der Gegenstand umströmt wird, kann eine entsprechend schnelle definierte Abkühlung erreicht werden.

Alternativ kann das Umströmen des aus dem Werkzeug entnommenen Gegenstandes mit dem Fluid zur langsamen Abkühlung genutzt werden. Dies kann zum Beispiel zur Beseitigung oder Verhinderung von Kühlspannungen beispielsweise bei amorphen Kunststoffen wünschenswert sein. Das verwendete Fluid kann entsprechend temperiert werden, um eine gezielte langsame Abkühlung des Gegenstandes zu erreichen. Durch entsprechende Temperierung und Steuerung des Volumenstromes des Fluids kann somit die Abkühlgeschwindigkeit des aus dem Werkzeug entnommen Gegenstandes je nach Art des verwendeten Kunststoffes bzw. Materials über einen breiten Bereich gezielt eingestellt werden.

Der Gegenstand wird vorzugsweise mit weiteren Bauteilen zusammengefügt. Dabei können sowohl der Gegenstand als auch gegebenenfalls die weiteren Bauteile in der beschriebenen Weise durch eine Fluidströmung vor Verunreinigungen aus der Umgebungsluft geschützt werden.

Insbesondere kann der Gegenstand ein Behälter, z.B. ein medizinischer Behälter sein, welcher mit weiteren Bauteilen zusammengefügt und/oder befüllt und verschlossen wird. Dabei können mehrere oder alle der zusammenzusetzenden Behälterteile in der zuvor beschriebenen Weise aus einem Werkzeug entnommen und gehandhabt werden. So können beispielsweise Spritzenkörper und Kappe einer vorzufüllenden Spritze entsprechend gehandhabt werden, so dass alle mit einem Arzneimittel in Kontakt kommenden Teile des Behälters bzw, einer vorfüllbaren Spritze während des gesamten Produktions- bzw. Handhabungsprozesses vor Verunreinigungen aus der Umgebung geschützt sind.

Zusätzlich können zumindest einzelne Verfahrensschritte in einer kontrollierten Umgebung von Klasse 1000 oder geringerer Reinheit stattfinden. Eine Reinraumumgebung von Klasse 100, wie sie beim Stand der Technik erforderlich ist, ist gemäß dem erfindungsgemäßen Verfahren nicht notwendig, da der zu handhabende Gegenstand bzw. das zu handhabende Behälterteil ständig durch das umspülende Fluid vor Verunreinigungen geschützt wird. Sauberere Reinraumklassen verschlechtern das Ergebnis natürlich nicht und können in denjenigen Verfahrenschritten zum Einsatz kommen, wo sie z.B. nach behördlichen Vorschriften verlangt sind.

Gemäß einer weiteren bevorzugten Ausführungsform der Erfindung findet direkt nach der Entnahme des Gegenstandes aus dem Werkzeug eine Silikonisierung des Gegenstandes statt. Eine solche Silikonisierung ist beispielsweise bei der Herstellung vorfüllbarer medizinischer Behälter erforderlich. Die Silikonisierung direkt im Anschluss an die Entnahme aus dem Werkzeug, wenn der Gegenstand vorzugsweise noch nicht vollständig ausgekühlt ist, hat den Vorteil, dass die Oberfläche des Gegenstandes bereits aktiviert ist. So ist bei Gegenständen aus Kunststoff keine zusätzliche Aktivierung vor der Silikonisierung erforderlich, wodurch das Herstellungsverfahren weiter vereinfacht und beschleunigt wird. Nach der Silikonisierung kann dann zusätzlich eine visuelle Kontrolle mit dem Auge oder automatisch mit einer Kamera durchgeführt werden, wobei gleichzeitig der einwandfreie Zustand des Gegenstandes als auch die Qualität der Silikonisierung überprüft werden können.

Ferner kann das den Gegenstand umströmende Fluid zusätzlich zur Beeinflussung der Oberflächeneigenschaften des Gegenstandes eingesetzt werden. So kann das Fluid und insbesondere das Gas so gewählt werden, dass es vorbestimmte Reaktionen mit der Oberflächenschicht des Gegenstandes eingeht, um bestimmte Oberflächeneigenschaften zu erzielen. Alternativ können entsprechende Hilfsstoffe dem Fluid beigemischt werden. Zusätzlich können durch die Fluidströmung Hilfs- und Reaktionsstoffe wieder entfernt werden.

Besonders bevorzugt wird das den Gegenstand umströmende Fluid zur Härtung und/oder Trocknung einer Oberflächenbeschichtung eingesetzt. Diese Oberflächenbeschichtung kann beispielsweise Silikon sein, welches in einem Silikonisierungsschritt aufgetragen worden ist. Das umströmende Gas, welches den Gegenstand vor Umgebungseinflüssen schützt, kann dabei die Trocknung bzw. Härtung des Silikons beschleunigen.

Die Erfindung betrifft ferner eine Vorrichtung zur Handhabung eines hochreinen Gegenstandes, insbesondere eines medizinischen Gegenstandes wie eines medizinischen Behälters oder Behälterteils. Die zur Handhabung dienende Handhabungseinrichtung weist dazu zumindest eine Düse zum Ausströmen eines Fluids auf. Dabei ist die Düse zum Ausströmen des Fluids derart angeordnet, dass ein in der Handhabungseinrichtung gehaltener Gegenstand von dem Fluid umströmt wird. Das bedeutet, zumindest eine Düse ist so angeordnet, dass diejenigen Teile des Gegenstandes, welche von der Umgebungsluft abgeschirmt werden sollen, vollständig und kontinuierlich von dem Fluid überströmt werden, so dass das Fluid eine Schutzschicht bzw. Schutzhülle um den Gegenstand bilden kann. Die genaue Anordnung und Anzahl der verwendeten Düsen hängt dabei von der Form des zu schützenden Gegenstandes ab.

Vorzugsweise ist die Handhabungseinrichtung ein Roboterarm mit einer Greifeinrichtung zum Fassen des Gegenstandes. Dabei ist die zumindest eine Düse in der Nähe der Greifeinrichtung angeordnet. So kann der Gegenstand möglichst direkt umströmt werden, so dass der von der Fluidströmung gebildete Mantel möglichst eng an dem Gegenstand anliegt. Auf diese Weise wird die Menge des erforderlichen Fluid reduziert und eine den Gegenstand eng umgebende definierte Atmosphäre beispielsweise aus einem hochreinen Gas geschaffen.

Ferner ist an der Handhabungseinrichtung vorzugsweise ein das ausströmende Fluid zumindest teilweise abdeckender Schutzschild angeordnet. Ein solcher Schutzschild dient dazu, bei Bewegung der Handhabungseinrichtung ein Verwehen bzw. Verdrängen des Fluids zu verhindern. Daher wird der Schutzschild vorzugsweise wenigstens in Bewegungsrichtung vor dem Fluidmantel und dem darin liegenden Gegenstand angeordnet. Weiter bevorzugt ist der Schutzschild als Glocke ausgebildet, welche den Gegenstand und die den Gegenstand umgebende Fluidströmung umhüllt, so dass der den Gegenstand schützende Fluidmantel auch bei schneller Bewegung der Handhabungseinrichtung aufrechterhalten werden kann.

Nachfolgend wird die Erfindung am Beispiel der Herstellung eines medizinischen Behälters anhand der beigefügten Figuren beschrieben. In diesen zeigt:
- Fig. 1: eine perspektivische Gesamtansicht eines ersten Verfahrensschrittes,
- Fig. 2: eine perspektivische Gesamtansicht eines zweiten Verfahrensschrittes,
- Fig. 3: eine perspektivische Gesamtansicht eines dritten Verfahrensschrittes,
- Fig. 4: eine Draufsicht auf eine Anordnung zum Umströmen eines zu schützenden Gegenstandes,
- Fig. 5: eine perspektivische Ansicht der Anordnung gemäß Fig. 4,
- Fig. 6: eine Draufsicht auf eine weitere Anordnung zum Umströmen eines zu schützenden Gegenstandes,
- Fig. 7: eine perspektivische Ansicht der Anordnung gemäß Fig. 6,
- Fig. 8: eine Schnittansicht und Draufsicht einer weiteren Anordnung zum Umströmen eines zu schützenden Gegenstandes,
- Fig. 9: eine teilweise geschnittene perspektivische Ansicht der Anordnung gemäß Fig. 8,
- Fig. 10 + 11: schematisch den Wechsel zweier Anordnungen zum Umströmen eines zu schützenden Gegenstandes,
- Fig. 12: eine Draufsicht auf eine weitere Anordnung zum Umströmen eines zu schützenden Gegenstandes,
- Fig. 13: eine perspektivische Ansicht der Anordnung gemäß Fig. 12,
- Fig. 14: eine perspektivische Gesamtansicht einer Anlage zum Erzeugen und zur Weiterverarbeitung eines hochreinen Gegenanstandes,
- Fig. 15: eine perspektivische Gesamtansicht einer weiteren Anlagen zur Erzeugung und Weiterverarbeitung eines hochreinen Gegenstandes und
- Fig. 16 und Fig. 17: Flussdiagramme, in denen der Ablauf der Herstellung einer Spritze bzw. eines medizinischen Behälters nach den Verfahren gemäß Fig. 1 bis 15 gezeigt ist.

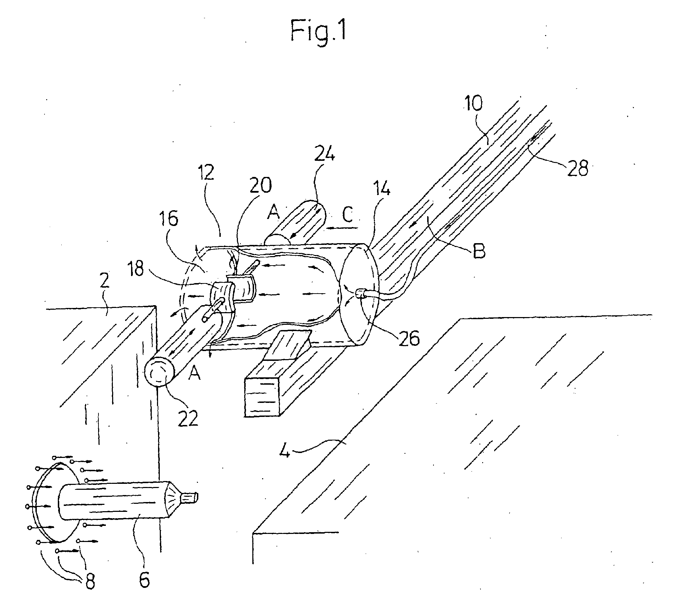

Anhand der Figuren 1 bis 3 wird schematisch ein bevorzugtes Ausführungsbeispiel des Entnahmevorganges eines Behälterteils aus einem Werkzeug gemäß der vorliegenden Erfindung beschrieben. Fig. 1 zeigt einen ersten Verfahrensschritt, in welchem die beiden Werkzeughälften 2 und 4 geöffnet sind. Das in dem Werkzeug 2, 4 gefertigte Behälterteil in Form einer Kunststoffspritze 6 befindet sich noch auf einem Kern an dem Werkzeug 2. Ringförmig umgebend zu dem Kern an dem Werkzeug 2 sind Düsen 8 angeordnet, durch welche Gas, vorzugsweise ionisierte und konditionierte hochreine Luft in Richtung der in Fig. 1 gezeigten Pfeile ausströmt. Das Ausströmen der Luft beginnt vorzugsweise mit Öffnen der Werkzeughälften 2 und 4. Die Strömungsrichtung verläuft so, dass die Luft möglichst linear in Längsrichtung an der Außenseite der Spritze 6 entlang strömt. Dadurch wird das Behälterteil, d. h. die Spritze 6 von einem Schutzmantel aus hochreiner Luft, welcher aus den Düsen 8 ausströmt, umgeben und somit vor Verunreinigungen aus der Umgebungsluft geschützt. Ferner bewirkt dieser Spülvorgang mit ionisierter Luft, dass möglicherweise beim Öffnen der Werkzeughälften 2 und 4 erzeugte statische Aufladungen in der Spritze 6 abgebaut werden. Auf diese Weise kann verhindert werden, dass sich Partikel aufgrund dieser statischen Aufladungen an den Spritzenoberflächen anlagern.

Ferner ist in Fig. 1 ein Roboterarm 10 gezeigt, an welchem eine Greifeinrichtung 12 zur Entnahme der Spritze 6 von der Werkzeughälfte 2 angebracht ist. Die Greifeinrichtung 12 besteht zunächst aus einer zylindrischen Glocke 14, welche an ihrer Vorderseite eine Öffnung 16 aufweist, durch welche die Spritze 6 aufgenommen werden kann. Im Bereich der vorderen, der Öffnung 16 zugewandten Endes der Glocke 14 sind zwei einander gegenüberliegende Greifer 18, 20 zum Halten der Spritze 6 angeordnet. Die Greifer 18 und 20 können über Stellantriebe 22, 24 linear in Richtung der Pfeile A bewegt werden, um die Spritze 6 zu ergreifen. Die Stellantriebe 22 und 24 können beispielsweise hydraulisch, pneumatisch oder elektrisch angetrieben sein. An ihren hinteren, der Öffnung 16 abgewandten Ende weist die Glocke 14 eine Gaseintrittsöffnung oder Düse 26 auf, welche über eine Leitung 28 mit einer Gasquelle, beispielsweise einer Luftaufbereitungseinrichtung in Verbindung steht. Vorzugsweise wird durch die Leitung 28 hochreine, ionisierte und konditionierte Luft durch die Gaseintrittsöffnung oder Düse 26 in Richtung der Pfeile in Fig. 1 in das Innere der Glocke 14 geleitet. Dabei strömt die Luft parallel zur Längsrichtung der Glocke 14 zu der Öffnung 16 und tritt durch diese ins Freie aus.

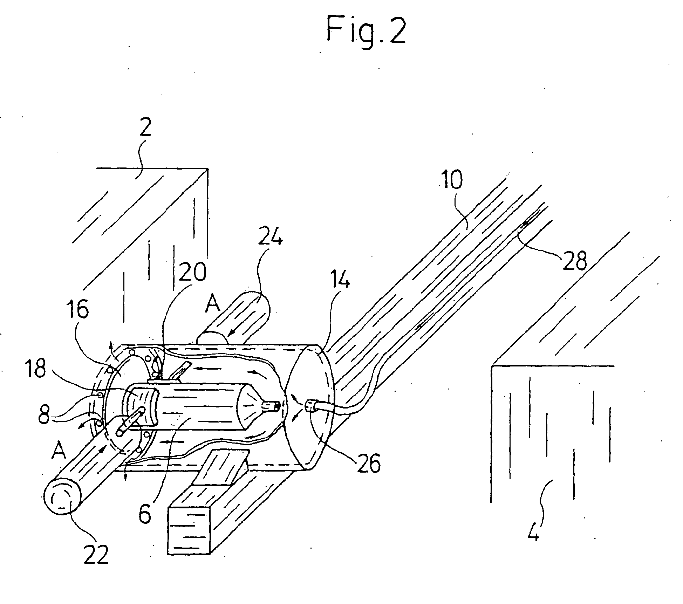

Zur Entnahme der Spritze 6 aus dem Werkzeug 2 wird der Roboterarm 10 zunächst in Richtung des Pfeils B bewegt, bis die Öffnung 16 der Glocke 14 gegenüberliegend zu der Spritze 6 angeordnet ist. Anschließend wird der Roboterarm 10 in Richtung des Pfeils C bewegt, so dass die Glocke 14 und die Greifer 16 und 18 über die Spritze 6 gestülpt werden, wie in Fig. 2 gezeigt ist. Die Glocke ist in Richtung des Pfeils C in Fig. 1 soweit bewegt worden, dass sie die Spritze 6 an dem Werkzeug 2 vollständig umschließt. Dabei gelangt die Spritze 6 zwischen die Greifer 18 und 20. Die Greifer 18 und 20 werden durch die Stellantriebe 22, 24 in Richtung der Pfeile A in Fig. 2 bewegt, so dass die Spritze 6 zwischen den Greifern 18 und 20 geklemmt wird. Gleichzeitig strömt kontinuierlich hochreine, ionisierte und konditionierte Luft durch die Gaseintrittsöffnung 26 in die Glocke 14 ein und strömt innerhalb der Glocke an der Außenseite der Spritze 6 entlang und tritt anschließend durch die Öffnung 16 an der Glocke 14 nach außen aus. Wenn die Glocke 14 die Spritze 6 in der in Fig. 2 gezeigten weise vollständig umgibt, kann der Gasstrom durch die Düsen 8 in dem Werkzeug 2 abgeschaltet werden, da die Spritze 6 in diesem Zustand durch den Gas- bzw. Luftstrom in der Glocke 14 vollständig umschlossen wird. Der Luftstrom in der Glocke 14, bewirkt, dass die Spritze 6 vollständig von der Umgebungsluft abgeschirmt wird und auf diese Weise vor Verschmutzungen aus der Umgebungsluft geschützt wird.

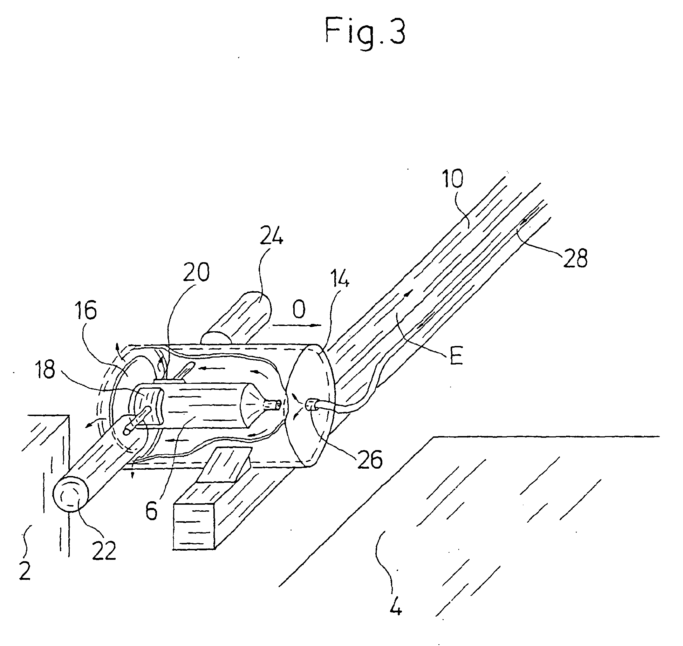

Nach dem Ergreifen der Spritze 6 durch die Greifer 18, 20 wird der Roboterarm in Richtung des Pfeil D in Fig. 3 von dem Werkzeug 2 wegbewegt. Gleichzeitig kann gegebenenfalls der Werkzeugeigene Auswerfer diese Bewegung unterstützen, so dass die punktuell auf die Spritze einwirkenden Kräfte gering bleiben. Dies erlaubt dann eine Entformung bei relativ hohen Temperaturen. Im Einzelfall kann aber durch den Greifer 18, 20 auch auf den Werkzeugauswerfer verzichtet werden. Dabei wird die Spritze 6, welche in der Glocke 14 durch die Greifer 18, 20 gehalten wird von einem Kern der Werkzeughälfte 2 abgezogen. Bei dieser Bewegung wird der Luftstrom in der Glocke 14 beibehalten, wie durch die Pfeile in Fig. 3 dargestellt ist. Das heißt, die Spritze 6 im Inneren der Glocke wird vollständig von hochreiner, ionisierter Luft umströmt und somit von der Umgebungsluft abgeschirmt. Das durch das Abziehen der Spritze entstehende Volumen wird mit gereinigter und konditionierter Luft gefüllt, so dass vor allem auch das Innere der Spritzen sauber bleibt und eine mögliche Aufladung bereits während Ihrer Entstehung neutralisiert wird. Gleichzeitig schützt die Glocke 14 bei schneller Bewegung des Roboterarms 10 davor, dass die Luftströmung verweht wird und der von der Luftströmung gebildete Schutzmantel um die Spritze 6 zerstört würde. Auf diese Weise kann die Spritze 6 bei der Bewegung und der Entnahme aus dem Werkzeug 2, 4 zuverlässig vor Verunreinigungen geschützt werden.

Im Anschluss an die Bewegung in Richtung des Pfeils D führt der Roboterarm 10 eine Bewegung in Richtung des Pfeils E in Fig. 3 aus, wodurch die Spritze 6 aus dem Raum zwischen den Werkzeughälften 2 und 4 entnommen wird. Im Anschluss kann die Spritze 6 von dem Roboterarm 10 in eine Weiterverarbeitung befördert werden, wo die Spritze beispielsweise silikonisiert, inspiziert, montiert, befüllt, verpackt etc. werden kann. Auch bei dieser Weiterverarbeitung verbleibt die Spritze im Roboterarm und/oder wird die Spritze 6 vorzugsweise über entsprechend Düsen mit hochreiner Luft umspült, um die Spritze vor Verunreinigungen zu schützen.

Die vorangehende Beschreibung bezieht sich lediglich auf eine bevorzugte Ausführungsform der Erfindung. Die Erfindung kann in vielfältigen Varianten ausgeführt werden. So kann beispielsweise auf die Glocke 14 an dem Roboterarm 10 verzichtet werden. Dabei werden die Greifer 18 und 20 sowie die Stellantriebe 12 und 14 direkt an dem Roboterarm 10 angeordnet. An dem Roboterarm 10 befinden sich entsprechende Luftdüsen, welche so angeordnet sind, dass eine von den Greifern 18 und 20 gehaltenes Bauteil, beispielsweise eine Spritze, auch ohne Glocke 14 vollständig mit Gas umspült werden kann, um es vor Verunreinigungen zu schützen.

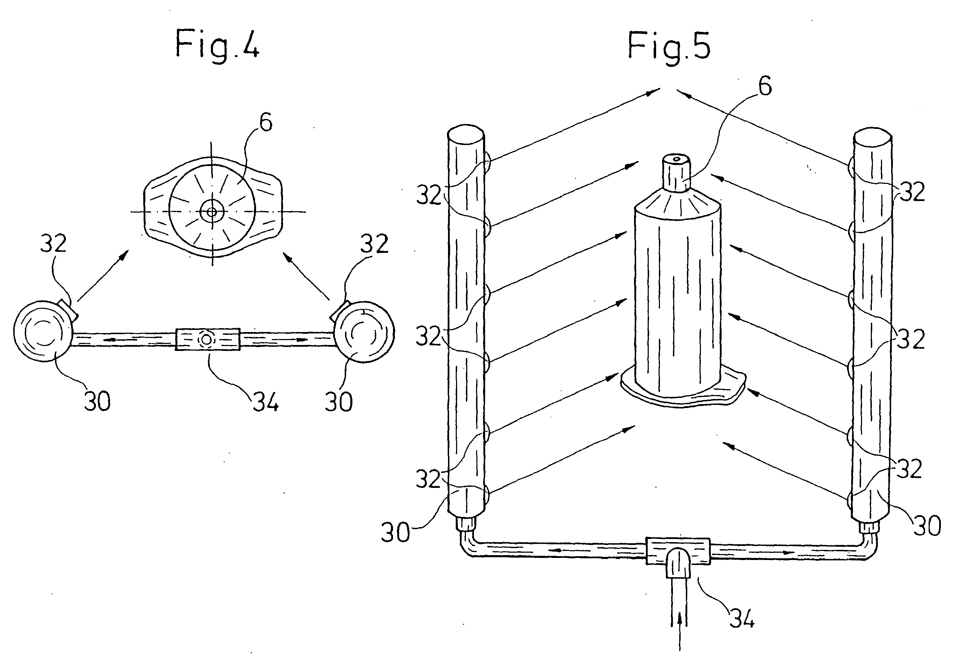

Anhand von Figuren 4 und 5 ist eine erste Anordnung zur Umströmung eines hochreinen Gegenstandes, im gezeigten Beispiel eine Spritze 6, gezeigt. Auch wenn das Beispiel sich auf die Handhabung einer Spritze 6 bezieht, so können doch auf gleiche Weise andere hochreine Bauteile gehandhabt werden. In Fig. 4 ist eine Draufsicht und in Fig. 5 eine perspektivische Ansicht der Anordnung zu sehen. Die Anordnung besteht aus zwei Düsenrohren 30, welche jeweils eine Vielzahl von Düsen 32 aufweisen. Die Düsenrohre 30 erstrecken sich im gezeigten Beispiel parallel zueinander und parallel zur Längsachse der Spritze 6. Über die gesamte Länge der Düsenrohre ist jeweils eine Reihe von Düsen 32 angeordnet, durch die ein Fluid bzw. Gas ausgeströmt wird, um die Spritze 6 zu umströmen und so von der Umgebung abzuschirmen. An einem Ende sind die Düsenrohre 30 mit einem Rohrleitungssystem 34 verbunden, durch welches das Fluid, insbesondere ein Gas, beispielsweise hochreine Luft in die Düsenrohre 30 eingeleitet wird. Die Fluidströmung ist in Figuren 4 und 5 durch Pfeile angedeutet. Dabei sind die Düsen 32 so ausgerichtet, dass die Strömung von zwei Seiten im Wesentlichen in einem Winkel von 90° zueinander auf die Spritze 6 gerichtet sind, so dass die Spritze 6 von dem Fluid von allen Seiten vollständig umströmt werden kann, und die Spritze 6 durch das Fluid ummantelt und von der Umgebungsluft abgeschirmt wird.

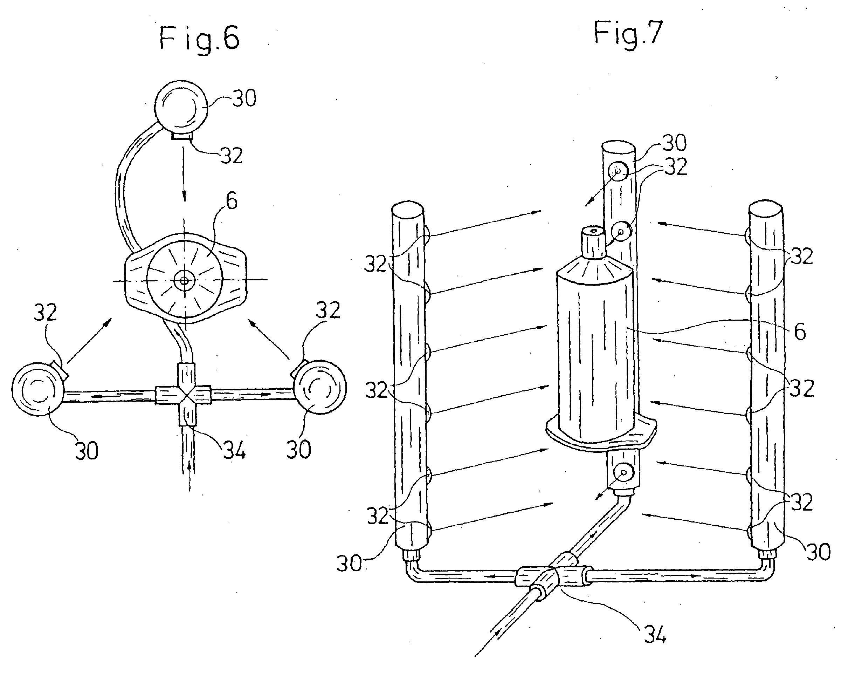

Figuren 6 und 7 zeigen eine Variante der Anordnung gemäß Figuren 4 und 5, wobei Fig. 6 eine Draufsicht und Fig. 7 eine perspektivische Ansicht der Anordnung zeigt. Im Unterschied zu der Anordnung gemäß Figuren 4 und 5 sind bei der Anordnung gemäß Figuren 6 und 7 drei Düsenrohre vorgesehen, welche gleichmäßig verteilt um den Umfang der zu schützenden Spritze 6 angeordnet sind, so dass die Spritze 6 von allen Seiten mit Fluid umströmt wird, wie in Figuren 6 und 7 durch die Pfeile angedeutet ist. Im Übrigen entspricht die Ausgestaltung der Düsenrohre 30 der anhand von Figuren 4 und 5 beschriebenen Ausgestaltung. Die drei Düsenrohre 30 sind mit einem Rohrleitungssystem 34 zur Versorgung mit einem Fluid bzw. Gas verbunden, wobei die Fluidströmung in dem Rohrleitungssystem 34 in Figuren 6 und 7 durch Pfeile dargestellt ist.

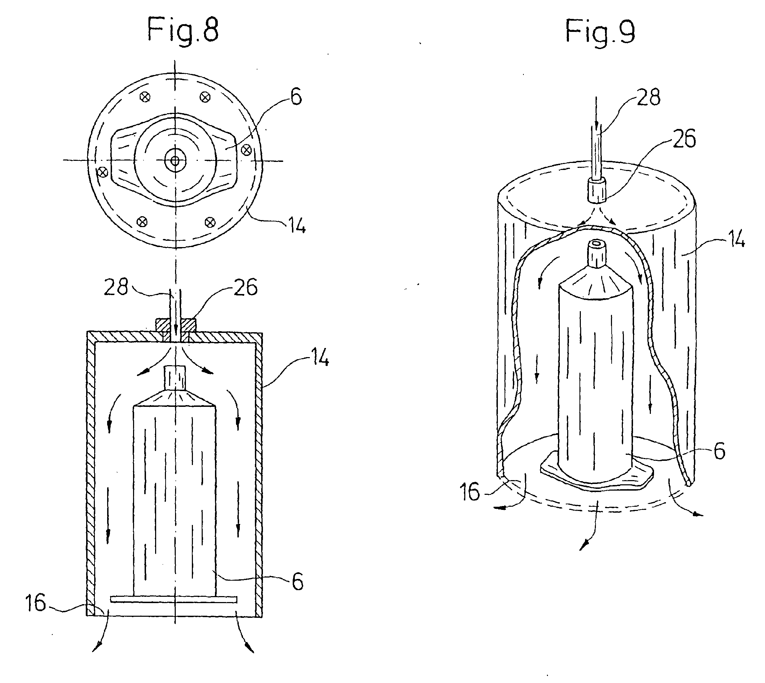

Figuren 8 und 9 zeigen eine weitere Anordnung zum Umströmen eines hochreinen Gegenstandes, im Beispiel einer Spritze 6, mit einem Fluid, beispielsweise einem Gas wie hochreiner Luft. In der Ausführungsform gemäß Figuren 8 und 9 wird die Spritze 6 von einer Glocke 14 umgeben. Fig. 8 zeigt eine Draufsicht und eine Schnittansicht dieser Anordnung, während Fig. 9 eine teilweise geschnittene perspektivische Ansicht zeigt. Die Glocke 14 ist zylindrisch ausgebildet und an einer Seite mit einer Öffnung 16 versehen, durch welche die Spritze 6 in die Glocke 14 eingesetzt werden kann bzw. die Glocke 14 über die Spritze 6 gestülpt werden kann. An der entgegengesetzten Stirnseite ist die Glocke 14 geschlossen und weist eine Gaseintrittsöffnung bzw. eine Düse 26 auf, welche mit einer Rohrleitung 28 zur Zufuhr eines Fluids bzw. Gases in Verbindung steht. Das Fluid strömt durch die Düse 26 in die Glocke 14 ein, wie durch die Pfeile in Figuren 8 und 9 angedeutet ist. Dabei strömt das Fluid über die Außenseiten der Spritze 6, so dass die Spritze 6 vollständig von dem Fluid umströmt wird, so dass das Fluid einen Schutzmantel um die Spritze 6 bildet. Anschließend tritt das Fluid durch die Öffnung 16 aus der Glocke 14 aus. Die Glocke 14 hat bei dieser Anordnung den Zweck, bei einer Bewegung der Spritze 6 ein Verwehen des umgebenden Fluids zu verhindern. Auf diese Weise kann gewährleistet werden, dass der Schutzmantel aus dem umströmenden Fluid auch bei schnellen Bewegungen aufrechterhalten wird.

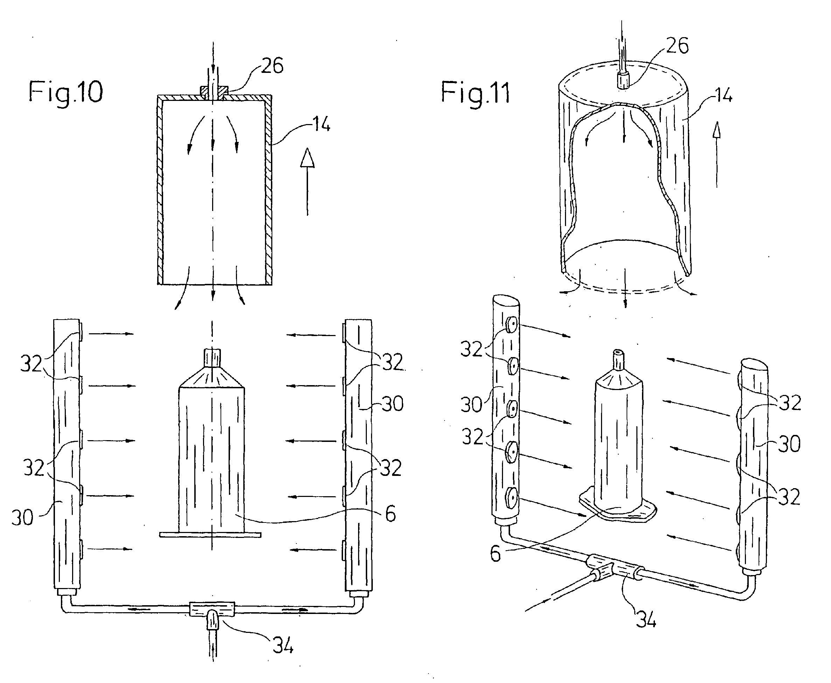

Anhand von Figuren 10 und 11 wird gezeigt, wie ein Gegenstand, im gezeigten Beispiel eine Spritze 6, aus einer Glocke 14 gemäß Figuren 8 und 9 in eine Anordnung gemäß Figuren 4 bis 7 transferiert werden kann. Dazu zeigt Fig. 10 eine teilweise geschnittene Seitenansicht und Fig. 11 eine teilweise geschnittene perspektivische Ansicht. Zunächst wird die Glocke 14 mit der darin angeordneten Spritze 6 (siehe Figuren 8 und 9) in eine Position zwischen den Düsenrohren 30 gebracht. In Figuren 10 und 1 ist eine Anordnung mit zwei Düsenrohren 30 gezeigt. Es kann jedoch auch eine Anordnung von weniger oder mehr Düsenrohren, beispielsweise drei Düsenrohren, wie anhand von Figuren 6 und 7 erläutert, vorgesehen sein. Anschließend wird die Glocke 14 angehoben, wobei die Spritze 6 zwischen den Düsenrohren 30 verbleibt. Aus den Düsenrohren 30 strömt durch deren Düsen 32 dabei wie aus der Düse 26 in der Glocke 14 das schützende Fluid aus, so dass die Spritze 6 auch beim Anheben der Glocke 14 vollständig von einem Fluid umströmt wird. Wenn die Glocke 14 entfernt ist, ist die Spritze 6 frei zugänglich für weitere Verarbeitungsschritte, beispielsweise eine Markierung oder Inspizierung oder Montage sowie sämtliche Arbeiten an den äußeren Oberflächen. Dabei wird jedoch weiterhin durch das aus den Düsen 32 der Düsenrohre ausströmende Fluid ein schützender Fluidmantel um die Spritze 6 herum aufrechterhalten, so dass eine Verunreinigung der Spritze 6 durch die Umgebungsluft verhindert werden kann. Auch in Figuren 10 und 1 ist die Fluidströmung durch Pfeile angedeutet.

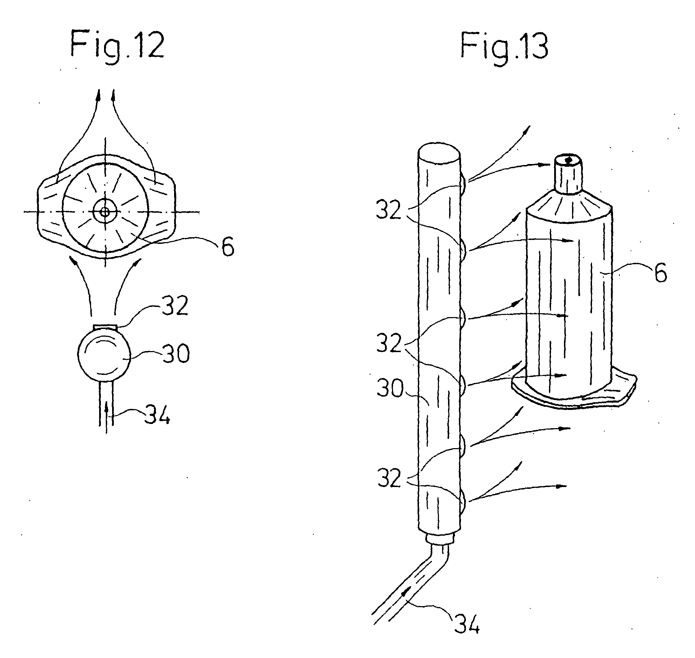

Figuren 12 und 13 zeigen eine Anordnung ähnlich den Figuren 4 bis 7, wobei jedoch lediglich ein Düsenrohr 30 vorgesehen ist. Das Düsenrohr 30 erstreckt sich im Wesentlichen parallel zur Längsachse der Spritze 6, so dass die Düsen 32 der Spritze 6 zugewandt sind. Das ausströmende Fluid umströmt dabei, wie in Fig. 12 in der Draufsicht dargestellt ist, die Spritze 6 derart, dass die Strömung an der Rückseite der Spritze 6, d. h. an der dem Düsenrohr 30 abgewandten Seite der Spritze 6, wieder zusammengeführt wird, so dass ein geschlossener Fluidmantel gebildet wird, welcher die Spritze 6 von allen Seiten schützend umschließt. Eine solche Anordnung ist hauptsächlich für einen Gegenstand wie eine Spritze 6 mit rundem Querschnitt geeignet, welcher ein Zusammenfließen der Strömung an der Rückseite der Spritze 6 ermöglicht. Je nach Form und Größe des zu schützenden Gegenstandes müssen unterschiedliche Arten und Anzahlen von Düsen 32 oder Düsenrohren 30 am Umfang des Gegenstandes angeordnet werden, um einen den Gegenstand vollständig umgebenden Fluidmantel zu erzeugen.

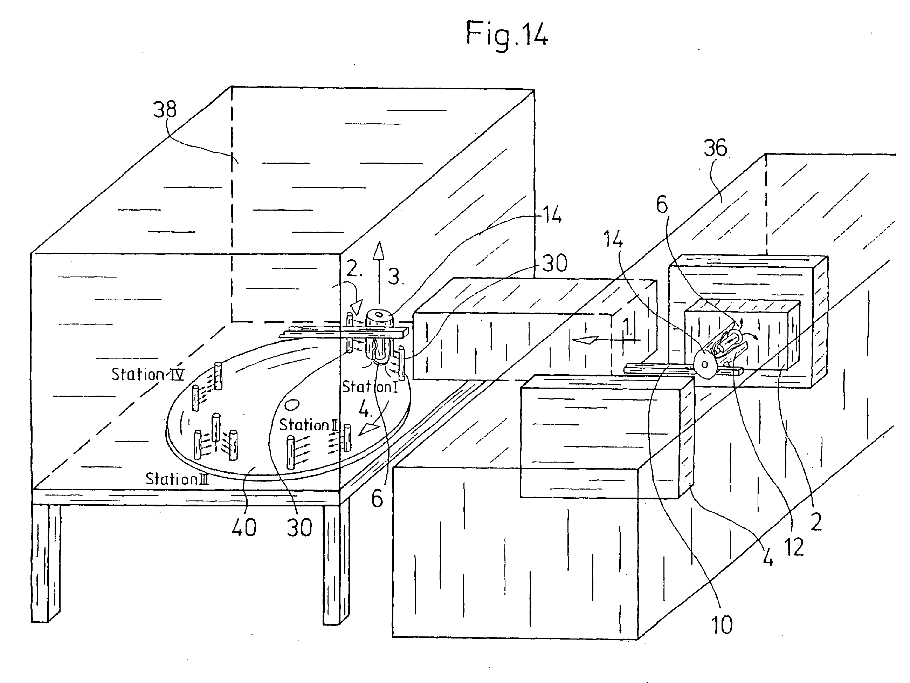

Fig. 14 zeigt eine schematische Gesamtansicht einer Anlage zur Herstellung und Verarbeitung eines hochreinen Gegenstandes. Das gezeigte Beispiel betrifft eine Anlage zur Erzeugung eines medizinischen Behälters wie einer Spritze 6. Die Anlage besteht im Wesentlichen aus einer Spritzgussmaschine 36 und einer Weiterverarbeitungsanlage 38. Die Spritzgussmaschine 36 weist zwei Werkzeughälften 2 und 4 auf, aus denen die Spritze 6, wie anhand der Figuren 1 bis 3 erläutert ist, durch einen Roboterarm 10 mit einer Greifeinrichtung 12 und einer Glocke 14 entnommen wird. Dabei wird die Spritze 6 ständig von einem Gas umströmt, um die Sprite 6 von Vorverunreinigungen aus der Umgebungsluft zu schützen. Anschließend wird die Spritze 6 in der Glocke 14 bei ständiger Umströmung durch das Gas von dem Roboterarm 10 in die Weiterverarbeitungseinrichtung 38 transferiert, wie durch den Pfeil 1 in Fig. 14 angedeutet ist. Die Weiterverarbeitungsanlage 38 kann ein geschlossenes System sein, in dem definierte Umgebungsbedingungen herrschen. In der Weiterverarbeitungsanlage 38 wird bei der Station 1 die Spritze 6 aus der Glocke 14 in eine Anordnung gemäß Figuren 4 bis 7 oder Figuren 12 und 13 transferiert, wie anhand von Figuren 8 und 9 näher erläutert worden ist. Die Anordnung der Düsenrohre und eine, hier nicht näher erläuterte Halterung für die Spritze 6 sind auf einem Karussell 40 angeordnet, welche die Spritze 6 gemeinsam mit den Düsenrohren 30 durch Drehung in Richtung des Pfeils 4 zu den Stationen II, III und IV weiter fördert. Die Anzahl der erforderlichen Stationen hängt von den Verarbeitungsschritten während der Weiterverarbeitung ab. An den Stationen II, III und IV sind andere Anordnungen von Düsenrohren 30 gezeigt. Dies soll andeuten, dass je nach Einsatzzweck und Art des Gegenstandes unterschiedliche Anordnungen von Düsenrohren 30, beispielsweise gemäß den Figuren 4 bis 7 und 12 und 13 auf dem Karusseil 40 angeordnet werden können. Die weiteren Verarbeitungsschritte für die Spritze 6, können beispielsweise eine Silikonisierung, eine Kontrolle, eine Montage mit weiteren Spritzen bzw. Behälterteilen und/oder ein Befüllen der Spritze 6 sein. Dazu wird die Spritze 6 durch Drehung des Karussells 40 von Station zu Station weiter gefördert, wo jeweils ein Verarbeitungsschritt durchgeführt wird. Dabei drehen sich die Düsenrohre 30 an der Spritze 6 mit dem Karussell 40 mit, so dass die Spritze 6 ständig von einem Fluid schützend umströmt werden kann. Auf diese Weise kann während der gesamten Weiterverarbeitung ein schützender Fluidmantel aufrechterhalten werden, welcher die Spritze 6 vor Verunreinigungen aus der Umgebung schützt.

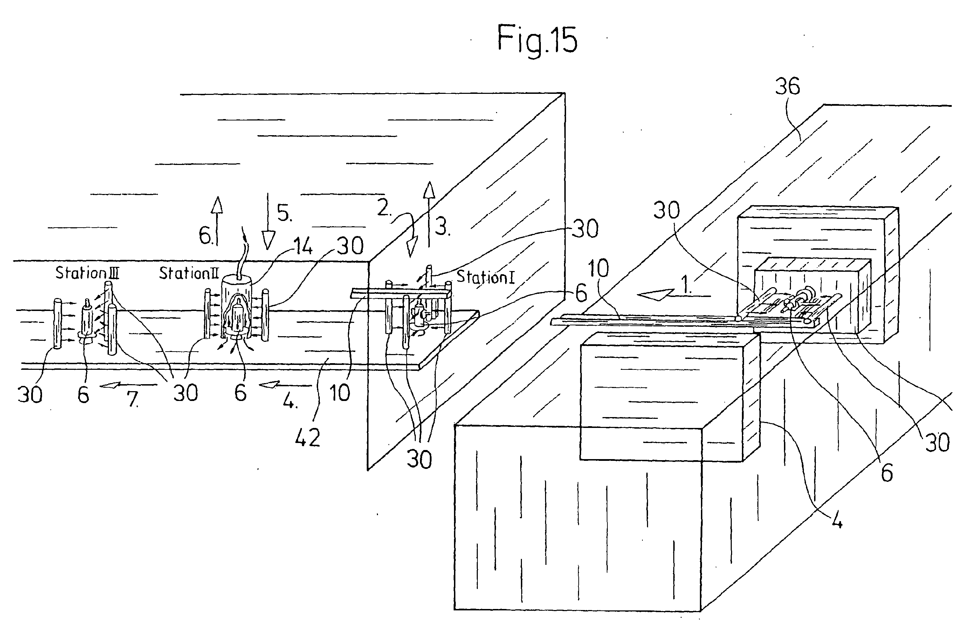

Fig. 15 zeigt eine alternative Anordnung zu Fig. 14. Die Anlage gemäß Fig. 15 ist ähnlich zu der gemäß Fig. 14. Die Spritzgussmaschine 36 entspricht der anhand von Fig. 14 beschriebenen Spritzgussmaschine. Im Unterschied zu der Anordnung gemäß Fig. 14 ist an dem Roboterarm 10 keine Glocke 14 angeordnet. Stattdessen sind an dem Roboterarm zwei Düsenrohre 30 mit Düsen 32 angeordnet, durch die das Fluid um die Spritze 6 geleitet wird, um einen schützenden Mantel zu bilden. Im Übrigen entspricht die Ausgestaltung der Greifeinrichtung 12, wie sie anhand von Figuren 1 bis 3 erläutert wurde. Die Spritze 6 wird gemäß obiger Beschreibung aus der Spritzgussmaschine 36 entnommen und in die Weiterverarbeitungsanlage 38 transferiert. Im Unterschied zu der Anordnung gemäß Fig. 14 ist bei dieser Anordnung in der Weiterverarbeitungsanlage 38 kein Karussell 40, sondern ein Lineartisch 42 angeordnet, durch welchen die Spritze 6 gemeinsam mit den umgebenden Düsenrohren von Station I zu Station II, zu Station III usw. transferiert wird, abhängig davon, wie viel Verarbeitungsstationen vorgesehen sind. An den Verarbeitungsstationen werden unterschiedliche Verarbeitungsschritte, beispielsweise Silikonisierung, Kontrolle, Montage, etc. vorgenommen. Dabei wird die Spritze 6 zwischen den Stationen immer gemeinsam mit den umgebenden und an dem Lineartisch 42 angeordneten Düsenrohren 30 bewegt, so dass der schützende Fluidmantel stetig aufrechterhalten wird.

An der Station 1 wird die Spritze 6 zunächst von dem Roboterarm 10 zwischen die Düsenrohre 30 an dem Lineartisch 42 abgesetzt. Dieser Transfer erfolgt ähnlich zu dem anhand der Figuren 8 und 9 erläuterten Transfer, mit dem Unterschied, dass anstatt einer Glocke 14 an dem Roboterarm 10 ebenfalls Düsenrohre 30 angeordnet sind. Die Düsenrohre 30 an dem Roboterarm 10 greifen dabei zwischen die Düsenrohre 30 an dem Lineartisch 32, so dass die Spritze 6 ständig von Fluid umströmt werden kann. Anstelle der Düsenrohre 30 an dem Roboterarm 10 kann auch bei dieser Anordnung eine Glocke 14 vorgesehen werden, wie bei Station II als alternative Ausführungsform angedeutet ist. Dabei würde der Transfer zwischen die Düsenrohre 30, wie anhand von Figuren 8 und 9 erläutert, erfolgen. Ferner können unterschiedliche Anzahlen von Düsenrohren 30 an den jeweiligen Aufnahmepositionen für eine Spritze 6 angeordnet werden, wie durch die unterschiedlichen Anordnungen bei Station I, Station II und Station III gezeigt ist. Die Anzahlen der Düsenrohre hängt von der Geometrie der Spritze 6 bzw. eines zu schützenden Gegenstandes und dem auszuführenden Verarbeitungsschritt ab. Die Anordnung wird immer so gewählt, dass der Gegenstand bzw. die Spritze 6 ausreichend durch das umgebende Fluid vor Verunreinigungen geschützt werden kann. Im gezeigten Beispiel in Figuren 14 und 15 sind an den einzelnen Stationen unterschiedliche Anordnungen von Düsenrohren 30 zur Veranschaulichung verschiedener Ausführungsformen gezeigt. Tatsächlich jedoch wird die Spritze 6 in derselben Anordnung von Düsenrohren 30 durch das Karussell 40 bzw. den Lineartisch 42 von Station zu Station gefördert wie durch den Pfeil 4 und den Pfeil 7 angedeutet ist.

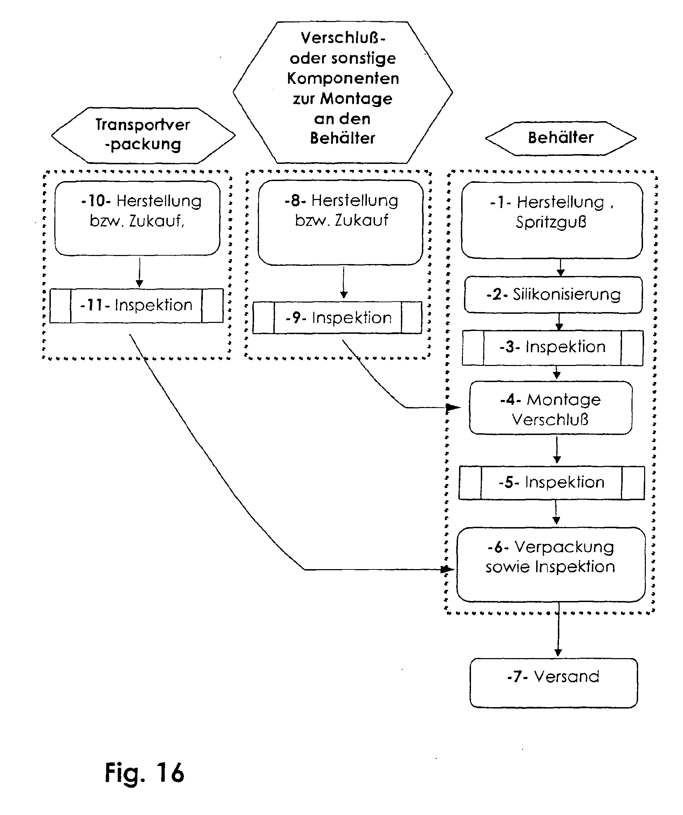

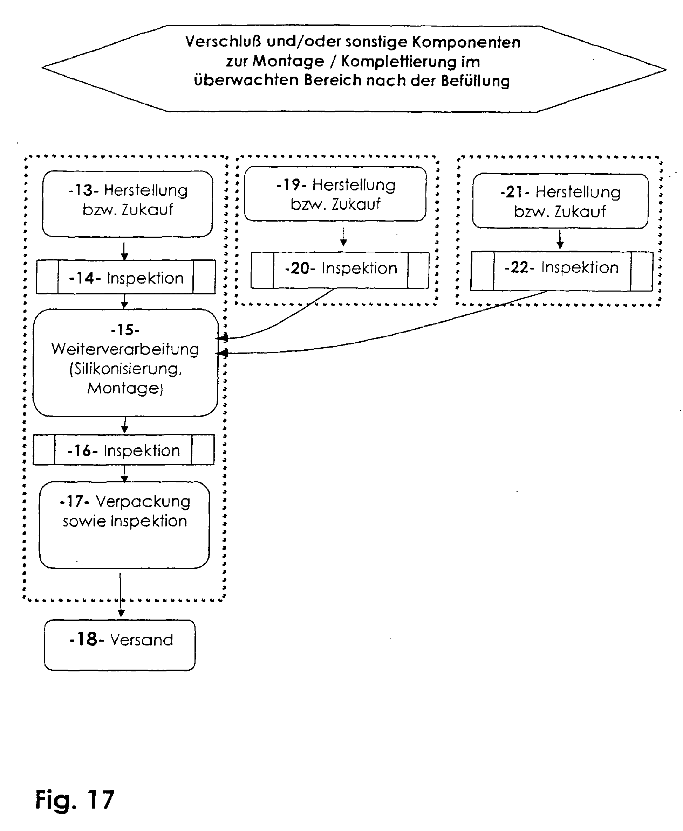

Figuren 16 und 17 zeigen in Flussdiagrammen noch einmal den Ablauf des vorangehend beschriebenen Verfahrens. Dabei ist in den Flussdiagrammen nicht nur die Herstellung des Gegenstandes bzw. der Spritze 6 sondern auch die Herstellung und Montage sämtlicher Zubehörteile sowie der Verpackung beschrieben. Die Verfahrensschritte 1 bis 7 in Fig. 16 beziehen sich unmittelbar auf die Herstellung der Spritze bzw. des Behälters 6. Im Verfahrensschritt 1 wird der Behälter bzw. die Spritze im Spritzgussverfahren hergestellt. Dabei wird aufgrund der beim Gießen herrschenden hohen Temperaturen ein keimfreier, hochfreier Gegenstand erzeugt. Bei der Entnahme aus dem Werkzeug hat der Gegenstand bzw. der Behälter je nach Art des verwendeten Kunststoffes vorzugsweise eine Temperatur zwischen 5° C und 150° C (PP/PE beispielsweise 15° C bis 100° C, PC beispielsweise 70° C bis 140° C, PET beispielsweise 5° C bis 60° C, PVC beispielsweise 20° C bis 85° C und COP beispielsweise 50° C bis 150° C). Im Verfahrenschritt 2 erfolgt dann eine Silikonisierung des gespritzten Behälters. Im Verfahrensschritt 3 schließt sich eine Inspektion bzw. Kontrolle an. Im Verfahrensschritt 4 wird dann ein Verschluss an dem Behälter montiert, welcher in den Verfahrensschritten 8 und 9, wie später beschrieben werden wird, gefertigt worden ist. Im Verfahrensschritt 5 schließt sich dann eine nochmalige Inspektion bzw. Kontrolle an, bevor dann im Verfahrensschritt 6 eine Primär- und Sekundär-Verpackung mit anschließender nochmaliger Inspektion erfolgt. Die Transportverpackung wird gemäß dem später beschriebenen Verfahrensschritten 10 und 11 hergestellt und im Verfahrensschritt 6 zugeführt. Als Verfahrensschritt 7 schließt sich dann der Versand des fertigen und verpackten Produktes an. Die Verfahrensschritte 1 und 6, welche in Fig. 16 von einer gepunkteten Linie umschlossen sind, finden sämtlich unter der oben beschriebenen Abschirmung des Gegenstandes bzw. Behälters 6 durch umströmende hochreine Luft statt. Es handelt sich dabei um eine lokale Luftströmung, welche direkt den zu bearbeitenden und zu handhabenden Behälter umströmt. Die Luft wird bevorzugt mit einem Druck zwischen 300 und 3500 hPa zugeführt. Dabei wird die Luft vor Zuführung zu dem zu umströmenden Gegenstand gefiltert. Der dazu eingesetzte Filter hat vorzugsweise eine Porengröße zwischen 0,1 und 3 µm und eine Abscheiderate deutlich über 99 %.

Der im Verfahrensschritt 4 an dem Behälter 6 montierte Verschluss wird im Verfahren 8 entweder ebenfalls im Spritzgussverfahren hergestellt oder als Zukaufsteil in den Prozess eingeführt. Dabei wird der Verschluss in hochreiner Form angeliefert oder, wie vorangehend am Beispiel des Behälters beschrieben, in hochreiner Form direkt aus der Spritzgussmaschine entnommen. Im Verfahrensschritt 9 schließt sich eine Inspektion bzw. Prüfung des Teiles an, bevor der Verschluss im Schritt 4 an dem Behälter montiert wird. Die Transportverpackung, in welcher der Behälter im Verfahrensschritt 6 verpackt wird, wird im Verfahrensschritt 10 dem Prozess zugeführt. Dabei wird die Verpackung entweder als Zukaufsteil in hochreiner, d. h. keimfreier bzw. keimarmer Form angeliefert oder direkt, wie oben anhand des Behälters beschrieben, aus einer Spritzgussmaschine entnommen. Auch die Verfahrensschritte 10 und 11 sowie 8 und 9 erfolgen jeweils in der Weise, dass der entsprechende Gegenstand durch hochreine Luft, welche den Gegenstand direkt umströmt, von der Umgebungsluft abgeschirmt wird, um ihn vor Verunreinigungen zu schützen. Dies ist in Fig. 16 durch die gepunkteten Linien angedeutet, d. h. die in den gepunkteten Linien dargestellten Verfahrensschritte werden unter Anwendung der erfindungsgemäßen Abschirmung, wie sie oben im Detail beschrieben wurde, durchgeführt.

Fig. 17 zeigt ein weiteres Flussdiagramm, in welchem die Herstellung eines Verschlusses und/oder sonstiger Komponente gezeigt ist, welche nach einer Befüllung an den Behälter, welcher gemäß dem Ablauf in Fig. 16 gefertigt wurde, montiert werden. Dieser Verschluss wird beispielsweise nach dem Befüllen in den Behälter bzw. die Spritze 6 eingesetzt und dient später bei Benutzung der Spritze als Kolben. In den Schritten 13, 19 und 21 werden entsprechende Teile des Verschlusses in den Prozess eingeführt. Dies kann entweder in Form von Zukaufteilen geschehen, welche in hochreiner Form angeliefert und in den Prozess eingeschleust werden. Alternativ können die Teile, wie vorangehend am Beispiel des Behälters beschrieben, heißgeformt und im noch warmen Zustand der Maschine entnommen werden. In diesem Zustand sind die Gegenstände aufgrund der hohen Verarbeitungstemperaturen hochrein, so dass sie direkt weiterverarbeitet werden können. In den Schritten 14, 20 und 22 schließt sich eine Inspektion bzw. Kontrolle der so gefertigten bzw. zugeführten Einzelteile an. Dabei erfolgt die Handhabung der Einzelteile jeweils unter Abschirmung durch die Gegenstände direkt umströmende hochreine Luft, wie vorangehend am Beispiel des Behälters bzw. der Spritze 6 beschrieben wurde. Eine Montage der Einzelteile erfolgt im Verfahrensschritt 15, wobei in diesem Verfahrensschritt die in den Verfahrensschritten 13, 19 und 21 zugeführten Bauteile zusammengeführt werden. Neben der Montage kann auch eine Silikonisierung der Bauteile, insbesondere des als Kolben dienenden Verschlusses erfolgen. Anschließend schließt sich im Schritt 16 eine weitere Inspektion an, bevor der so montierte Gegenstand bzw. Verschluss dann in Schritt 17 verpackt und nochmals inspiziert wird. Im Schritt 18 erfolgt dann der Versand dieses Teils, was bevorzugt gemeinsam mit dem Versand des Behälters gemäß Verfahrensschritt 7 in Fig. 16 erfolgt. Auch in Fig. 17 sind die Verfahrensschritte, in denen die Handhabung eines hochreinen Gegenstandes gemäß dem zuvor am Beispiel des Behälters bzw. der Spritze 6 beschriebenen Verfahren erfolgt, durch gepunktete Linien umgrenzt.

### Bezugszeichenliste

- 2, 4 -: Werkzeughälften
- 6 -: Spritze
- 8 -: Düsen
- 10 -: Roboterarm
- 12 -: Greifeinrichtung
- 14 -: Glocke
- 16 -: Öffnung
- 18, 20 -: Greifer
- 22,24 -: Stellantriebe
- 26 -: Gaseintrittsöffnung, Düse
- 28 -: Leitung
- 30: Düsenrohre
- 32: Düsen
- 34: Rohrleitungssystem
- 36: Spritzgussmaschine
- 38: Weiterverarbeitungsanlage
- 40: Karussell
- 42: Lineartisch

## Patentansprüche

1. Verfahren zur Handhabung eines in einem Werkzeug heißgeformten hochreinen Gegenstandes (6), bei welchem der anfänglich hochreine Gegenstand (6) während des gesamten Entnahmevorgangs aus dem Werkzeug und mindestens eines nachfolgenden Handhabungsvorgangs durch ein umströmendes Fluid von der Umgebung abgeschirmt wird, wobei bei der Handhabung des Gegenstandes zumindest eine Düse zum Ausströmen des Fluids mit dem Gegenstand mitgeführt wird.

2. Verfahren nach Anspruch 1, bei welchem der Gegenstand ein Teil eines medizinischen Behälters (6) oder ein medizinischer Behälter (6) ist.

3. Verfahren nach Anspruch 1, bei welchem das Fluid, von dem der Gegenstand (6) umströmt wird, ein Gas, insbesondere Luft oder gefilterte Luft ist.

4. Verfahren nach einem der vorangehenden Ansprüche, bei welchem das Fluid, von dem der Gegenstand (6) umströmt wird, konditionierte Luft ist.

5. Verfahren nach einem der vorangehenden Ansprüche, bei welchem das Fluid, von dem der Gegenstand (6) umströmt wird, ionisierte Luft ist.

6. Verfahren nach einem der vorangehenden Ansprüche, bei welchem das Fluid, von dem der Gegenstand (6) umströmt wird, zumindest als Bestandteil ein keimtötendes Fluid bzw. Gas enthält.

7. Verfahren nach einem der Ansprüche 1 bis 6, bei welchem die Umströmung des Gegenstandes (6) mit dem Fluid beginnt, wenn sich der Gegenstand (6) noch in dem Werkzeug (2, 4) befindet.

8. Verfahren nach einem der Ansprüche 1 bis 7, bei welchem die Entnahme des Gegenstandes (6) aus dem Werkzeug (2, 4) in definierter Weise maschinell erfolgt.

9. Verfahren nach Anspruch 8, bei welchem der Gegenstand (6) aus dem Werkzeug (2, 4) durch einen Roboter (10) entnommen wird und gleichzeitig durch einen in dem Werkzeug (2, 4) angeordneten Auswerfer von dem Werkzeug (2, 4) getrennt wird.

10. Verfahren nach Anspruch 8 oder 9, bei welchem die Entnahme des Gegenstandes (6) aus dem Werkzeug (2, 4) mit einer geringen Anfangsgeschwindigkeit erfolgt.

11. Verfahren nach einem der Ansprüche 1 bis 10, bei welchem die Entnahme des Gegenstandes (6) aus dem Werkzeug (2, 4) vor dem vollständigen Abkühlen erfolgt.

12. Verfahren nach einem der Ansprüche 1 bis 11, bei welchem die Entnahme des Gegenstandes aus dem Werkzeug (2, 4) durch einen Roboter (10) erfolgt und an dem Roboter (10) die zumindest eine Düse (8) angeordnet ist, durch welche der Gegenstand (6) mit dem Fluid umströmt wird.

13. Verfahren nach einem der Ansprüche 1 bis 12, bei welchem in zumindest einem Teil (2) des Werkzeuges Düsen (8) zum Umströmen des Gegenstandes (6) mit dem Fluid angeordnet sind.

14. Verfahren nach einem der Ansprüche 1 bis 13, bei welchem das Werkzeug (2, 4) eine Oberfläche aufweist, welche derart behandelt ist, dass sie ein minimales Haftvermögen aufweist.

15. Verfahren nach einem der Ansprüche 1 bis 14, bei welchem der Gegenstand (6) direkt bei der Entnahme aus dem Werkzeug (2, 4) zusätzlich zu der Umströmung mit dem Fluid von einer Schutzglocke (14) umgeben wird.

16. Verfahren nach einem der Ansprüche 1 bis 15, bei welchem sich an die Entnahme des Gegenstandes (6) aus dem Werkzeug (2, 4) eine automatische oder halbautomatische Weiterverarbeitung anschließt.

17. Verfahren nach einem der Ansprüche 1 bis 16, bei welchem das Umströmen des aus dem Werkzeug (2, 4) entnommenen Gegenstandes (6) mit dem Fluid zur raschen Abkühlung des Gegenstandes (6) genutzt wird.

18. Verfahren nach einem der Ansprüche 1 bis 17, bei welchem das Umströmen des aus dem Werkzeug (2, 4) entnommenen Gegenstandes (6) mit dem Fluid zur langsamen Abkühlung genutzt wird.

19. Verfahren nach einem der vorangehenden Ansprüche, bei welchem der Gegenstand (6) mit weiteren Bauteilen zusammengefügt wird.

20. Verfahren nach einem der vorangehenden Ansprüche, bei welchem der Gegenstand (6) ein Behälter ist, welcher mit weiteren Bauteilen zusammengefügt wird und/oder befüllt und verschlossen wird.

21. Verfahren nach einem der vorangehenden Ansprüche, bei welchem zumindest einzelne Verfahrensschritte in einer kontrollierten Umgebung von Klasse 1000 oder geringerer Reinheit stattfinden.

22. Verfahren nach einem der Ansprüche 1 bis 21, bei welchem direkt nach der Entnahme des Gegenstandes (6) aus dem Werkzeug (2, 4) eine Silikonisierung des Gegenstandes (6) stattfindet.

23. Verfahren nach einem der vorangehenden Ansprüche, bei welchem das umströmende Fluid zur Beeinflussung der Oberflächeneigenschaften des Gegenstandes (6) eingesetzt wird.

24. Verfahren nach Anspruch 23, bei welchem das umströmende Fluid zur Härtung und/oder Trocknung einer Oberflächenbeschichtung eingesetzt wird.

25. Vorrichtung zur Entnahme aus dem Werkzeug und Handhabung eines in einem Werkzeug heißgeformten hochreinen Gegenstandes (6) mittels einer Handhabungseinrichtung (10), **dadurch gekennzeichnet, dass** an der Handhabungseinrichtung (10) zumindest eine Düse (26) zum Ausströmen eines Fluids derart angeordnet ist, dass ein in der Handhabungseinrichtung (10) befindlicher Gegenstand (6) von dem Fluid umströmt wird.

26. Vorrichtung nach Anspruch 25, bei welcher die Handhabungseinrichtung ein Roboterarm (10) mit einer Greifeinrichtung (12) ist, wobei die zumindest eine Düse (26) in der Nähe der Greifeinrichtung (12) angeordnet sind.

27. Vorrichtung nach Anspruch 25 oder 26, bei welcher an der Handhabungseinrichtung (10) ferner ein das ausströmende Fluid zumindest teilweise abdeckender Schutzschild (14) angeordnet ist.

## Claims

1. A method for handling a highly pure object (6) hot-moulded in a tool, with which the initially highly pure object (6) during the complete removal procedure from the tool and at least the subsequent handling procedure, is shielded from the surroundings by way of a fluid flowing around it, wherein with the handling of the object, at least one nozzle for the discharge of the fluid is led along with the object.

2. A method according to claim 1, with which the object is a part of a medical receptacle (6) or is a medical receptacle (6).

3. A method according to claim 1, with which the fluid which flows around the object (6) is a gas, in particular air or filtered air.

4. A method according to one of the preceding claims, with which the fluid which flows around the object (6), is conditioned air.

5. A method according to one of the preceding claims, with which the fluid which flows around the object (6), is ionised air.

6. A method according to one of the preceding claims, with which the fluid which flows around the object (6), at least as a share, contains a germicidal fluid or gas.

7. A method according to one of the claims 1 to 6, with which the circumflow of the object (6) with the fluid begins when the object (6) is still located in the tool (2, 4).

8. A method according to one of the claims 1 to 7, with which the removal of the object (6) from the tool (2, 4) is effected by machine in a defined manner.

9. A method according to claim 8, with which the object (6) is removed from the tool (2, 4) by way of a robot (10) and simultaneously is separated from the tool (2, 4) by way of an ejector arranged in the tool (2, 4).

10. A method according to claim 8 or 9, with which the removal of the object (6) from the tool (2, 4) is effected with a low initial speed.

11. A method according to one of the claims 1 to 10, with which the removal of the object (6) from the tool (2, 4) is effected before the complete cooling.

12. A method according to one of the claims 1 to 11, with which the removal of the object from the tool (2, 4) is effected by a robot (10), and at least one nozzle (8) is arranged on the robot (10), by way of which the object (6) is circumflowed by the fluid.

13. A method according to one of the claims 1 to 12, with which nozzles (8) for circumflowing the object (6) with the fluid are arranged in at least one part (2) of the tool.

14. A method according to one of the claims 1 to 13, with which the tool (2, 4) has a surface which is treated in a manner such that it has a minimal adhesion capability.

15. A method according to one of the claims 1 to 14, with which the object (6), additionally to the circumflow by the fluid directly on removal from the tool (2, 4), is surrounded by a protective bell (14).

16. A method according to one of the claims 1 to 15, with which an automatic or semi-automatic further processing follows the removal of the object (6) from the tool (2, 4).

17. A method according to one of the claims 1 to 16, with which the circumflow, by the fluid, of the object (6) removed from the tool (2, 4), is used for the rapid cooling of the object (6).

18. A method according to one of the claims 1 to 17, with which the circumflow, by the fluid, of the object (6) removed from the tool (2, 4), is used for slow cooling.

19. A method according to one of the preceding claims, with which the object (6) is joined together or assembled with further components.

20. A method according to one of the preceding claims, with which the object (6) is a receptacle which is assembled with further components and/or filled and closed.

21. A method according to one of the preceding claims, with which at least individual method steps take place in a controlled surrounding of Class 1000 or lesser purity.

22. A method according to claims 1 to 21, with which a siliconisation of the object (6) is effected directly after removal of the object (6) from the tool (2, 4).

23. A method according to one of the preceding claims, with which the circumflowing fluid is used for influencing the surface characteristics of the object (6).

24. A method according to claim 23, with which the circumflowing fluid is used for curing and/or drying a surface coating.

25. A device for removal from the tool and handling a highly pure object (6) which is hot-moulded in a tool, by way of a handling device (10), **characterised in that** at least one nozzle (26) for the discharge of a fluid is arranged on the handling device (10), in a manner such that an object (6) located in the handling means (10) is circumflowed by the fluid.

26. A device according to claim 25, with which the handling device is a robot arm (10) with a gripper means (12), wherein the at least one nozzle (26) is arranged in the vicinity of the gripper means (12).

27. A device according to claim 25 or 26, with which furthermore a protective shield (14) at least partly covering the fluid flowing out is arranged on the handling device (10).

## Revendications

1. Procédé pour la manipulation d'un objet (6) à très haut degré de propreté, façonné à chaud dans un outil, suivant lequel, pendant la phase entière de retrait de l'outil et au moins une phase de manipulation qui suit, l'objet (6), initialement à un très haut degré de propreté, est isolé de l'environnement par un fluide en écoulement enveloppant, au moins une buse, destinée à faire jaillir le fluide, étant alors entraînée avec l'objet lors de la manipulation de l'objet.

2. Procédé selon la revendication 1, dans lequel l'objet est une partie d'un récipient à usage médical (6) ou un récipient à usage médical (6).

3. Procédé selon la revendication 1, dans lequel le fluide, par l'écoulement duquel l'objet (6) est enveloppé, est un gaz, en particulier de l'air ou de l'air filtré.

4. Procédé selon l'une des revendications précédentes, dans lequel le fluide, par l'écoulement duquel l'objet (6) est enveloppé, est de l'air conditionné.

5. Procédé selon l'une des revendications précédentes, dans lequel le fluide, par l'écoulement duquel l'objet (6) est enveloppé, est de l'air ionisé.

6. Procédé selon l'une des revendications précédentes, dans lequel le fluide, par l'écoulement duquel l'objet (6) est enveloppé, contient au moins, en tant que constituant, un fluide ou un gaz stérilisant.

7. Procédé selon l'une des revendications 1 à 6, suivant lequel l'enveloppement de l'objet (6) avec le fluide en écoulement débute quand l'objet (6) se trouve encore dans l'outil (2, 4).

8. Procédé selon l'une des revendications 1 à 7, suivant lequel le retrait de l'objet (6) de l'outil (2, 4) se fait mécaniquement, d'une manière définie.

9. Procédé selon la revendication 8, suivant lequel l'objet (6) est retiré de l'outil (2, 4) par un robot (10) et est simultanément détaché de l'outil (2, 4) par un éjecteur disposé dans l'outil (2, 4).

10. Procédé selon la revendication 8 ou 9, suivant lequel le retrait de l'objet (6) de l'outil (2, 4) se fait sous une faible vitesse initiale.

11. Procédé selon l'une des revendications 1 à 10, suivant lequel le retrait de l'objet (6) de l'outil (2, 4) se fait avant le refroidissement complet.

12. Procédé selon l'une des revendications 1 à 11, suivant lequel le retrait de l'objet de l'outil (2, 4) se fait au moyen d'un robot (10), et sur le robot (10) est disposée la au moins une buse (8), par laquelle l'objet (6) est enveloppé avec le fluide en écoulement.

13. Procédé selon l'une des revendications 1 à 12, suivant lequel dans au moins une partie (2) de l'outil sont disposées des buses (8) pour l'enveloppement de l'objet (6) avec le fluide en écoulement.

14. Procédé selon l'une des revendications 1 à 13, suivant lequel l'outil (2, 4) présente une surface libre qui est traitée d'une manière telle, qu'elle présente un pouvoir d'adhérence minimal.

15. Procédé selon l'une des revendications 1 à 14, suivant lequel l'objet (6), directement au moment du retrait de l'outil (2, 4), est, en plus de son enveloppement avec le fluide en écoulement, entouré par une cloche de protection (14).

16. Procédé selon l'une des revendications 1 à 15, suivant lequel au retrait de l'objet (6) de l'outil (2, 4) succède immédiatement un traitement complémentaire automatique ou semi-automatique.

17. Procédé selon l'une des revendications 1 à 16, suivant lequel l'enveloppement de l'objet (6) retiré de l'outil (2, 4), avec le fluide en écoulement, est exploité pour le refroidissement rapide de l'objet (6).

18. Procédé selon l'une des revendications 1 à 17, suivant lequel l'enveloppement de l'objet (6) retiré de l'outil (2, 4), avec le fluide en écoulement, est exploité pour le refroidissement lent.

19. Procédé selon l'une des revendications précédentes, suivant lequel l'objet (6) est assemblé avec d'autres composants.

20. Procédé selon l'une des revendications précédentes, suivant lequel l'objet (6) est un récipient, qui est assemblé avec d'autres composants et/ou est rempli et fermé.

21. Procédé selon l'une des revendications précédentes, suivant lequel au moins quelques étapes du procédé se déroulent dans un environnement contrôlé de classe 1 000 ou d'une pureté moindre.

22. Procédé selon l'une des revendications 1 à 21, suivant lequel juste après le retrait de l'objet (6) de l'outil (2, 4), un siliconage de l'objet (6) a lieu.

23. Procédé selon l'une des revendications précédentes, suivant lequel le fluide en écoulement enveloppant est utilisé pour influer sur les propriétés superficielles de l'objet (6).

24. Procédé selon la revendication 23, suivant lequel le fluide en écoulement enveloppant est utilisé pour le durcissement et/ou le séchage d'un revêtement superficiel.

25. Appareil pour retirer de l'outil et manipuler un objet (6) à très haut degré de propreté, façonné à chaud dans un outil, au moyen d'un dispositif de manipulation (10), **caractérisé en ce que** sur le dispositif de manipulation (10) est disposée au moins une buse (26) destinée à faire jaillir un fluide d'une manière telle, qu'un objet (6) se trouvant dans le dispositif de manipulation (10), soit enveloppé par le fluide en écoulement.

26. Appareil selon la revendication 25, dans lequel le dispositif de manipulation est un bras de robot (10) comportant un dispositif de préhension (12), la au moins une buse (26) étant disposée au voisinage du dispositif de préhension (12).

27. Appareil selon la revendication 25 ou 26, dans lequel sur le dispositif de manipulation (10) est disposé, en outre, un bouclier protecteur (14) couvrant, au moins partiellement, le fluide à faire jaillir.
